# EUROPÄISCHE PATENTSCHRIFT

(11) **EP 2 680 967 B1**
(45) Veröffentlichungstag und Bekanntmachung des Hinweises auf die Patenterteilung: **13.05.2015**
(21) Anmeldenummer: 12721722.2
(22) Anmeldetag: 23.02.2012
(51) Int. Cl.: B01J 31/00, C07F 11/00

(54) **METALLKOMPLEXE DES MOLYBDÄNS UND WOLFRAMS UND IHRE VERWENDUNG ALS PRÄKATALYSATOREN FÜR DIE OLEFINMETATHESE**
MOLYBDENUM AND TUNGSTEN METAL COMPLEXES AND USE THEREOF AS PRECATALYSTS FOR OLEFIN METATHESIS
COMPLEXES MÉTALLIQUES DE MOLYBDÈNE ET DE TUNGSTÈNE ET LEUR UTILISATION COMME PRÉCATALYSEURS POUR LA MÉTATHÈSE DES OLÉFINES

(30) Priorität: 28.02.2011 DE 102011012629
(43) Veröffentlichungstag der Anmeldung: 08.01.2014
(73) Patentinhaber: Studiengesellschaft Kohle MbH, 45470 Mülheim an der Ruhr (DE)
(72) Erfinder: FÜRSTNER, Alois, 45478 Mülheim an der Ruhr (DE); HEPPEKAUSEN, Johannes, 45470 Mülheim an der Ruhr (DE)
(74) Vertreter: Ricker, Mathias
(86) Internationale Anmeldenummer: PCT/DE2012/100047
(87) Internationale Veröffentlichungsnummer: WO 2012/116695

(56) Entgegenhaltungen:
- FOX, H. H.; LEE, J.-K.; PARK, L. Y.; SCHROCK, R. R.: "SYNTHESIS OF FIVE-AND SIX-COORDINATE ALKYLIDENE COMPLEXES OF THE TYPE MO(CHR)(NAR)[OCME(CF3)2 ]Sx AND THEIR USE AS LIVING ROMP INITIATORS OR WITTIG REAGENTS", ORGANOMETALLICS, Bd. 12, 1993, Seiten 759-768, XP000564438, in der Anmeldung erwähnt
- JOHANNES HEPPEKAUSEN ET AL: "Rendering Schrock-type Molybdenum Alkylidene Complexes Air Stable: User-Friendly Precatalysts for Alkene Metathesis", ANGEWANDTE CHEMIE (INTERNATIONAL ED. IN ENGLISH), Bd. 123, Nr. 34, 16. August 2011 (2011-08-16), Seiten 7975-7978, XP55030857, ISSN: 0044-8249, DOI: 10.1002/ange.201102012
- JOHANNES HEPPEKAUSEN ET AL: "Practical New Silyloxy-Based Alkyne Metathesis Catalysts with Optimized Activity and Selectivity Profiles", JOURNAL OF THE AMERICAN CHEMICAL SOCIETY, Bd. 132, Nr. 32, 18. August 2010 (2010-08-18), Seiten 11045-11057, XP55002082, ISSN: 0002-7863, DOI: 10.1021/ja104800w
- KAREN M. TOTLAND, ET AL: 'Ring Opening Metathesis Polymerization with Binaphtholate or Biphenolate Complexes of Molybdenum' Bd. 20, Nr. 19, 09 September 1996, Seiten 6114 - 6125

## Beschreibung

Die vorliegende Erfindung betrifft Metallkomplexe des Molybdäns oder Wolframs, Verfahren zu ihrer Herstellung und ihre Verwendung als Präkatalysatoren für Olefinmetathese-Reaktionen.

Unter Alkenmetathese versteht man die wechselseitige Umalkylidenierung von Alkenen gemäß Schema 1 (Ivin, K. J.; Mol, J. C. Olefin Metathesis and Metathesis Polymerization, Academic Press, San Diego, 1997).

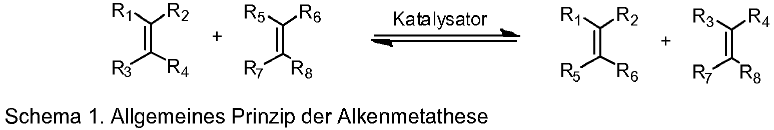

Alkenmetathesen werden durch Metallverbindungen katalysiert, die im Reaktionsgemisch in homogener oder heterogener Form vorliegen können. Als eigentlich katalytisch aktive Spezies gelten sogenannte Schrock-Alkylidene, die in situ oder ex situ dargestellt werden können (Schrock, R. R. Chem. Rev. 2002, 102, 145). Neben Alkylidenkomplexen des Rutheniums werden häufig Schrock-Alkylidene des Molybdäns und Wolframs benutzt, die sich durch hohe oder sehr hohe katalytische Aktivität auszeichnen (Schrock, R. R., Chem. Rev. 2009, 109, 3211; Schrock, R. R.; Hoveyda, A. H. Angew. Chem. Int. Ed. 2003, 42, 4592); einige Schrock-Alkylidene des Molybdäns und Wolframs sind auch kommerziell erhältlich. Im Gegensatz zu Ruthenium-Alkylidenen (Vougioukalakis, G. C.; Grubbs, R. R. Chem. Rev. 2010, 110, 1746) sind Schrock-Alkylidene des Molybdäns und Wolframs jedoch in der Regel sehr empfindlich gegenüber Luft und Feuchtigkeit und müssen daher unter inerten Bedingungen gehandhabt und umgesetzt werden. Durch Luft und Feuchtigkeit werden sie innerhalb kurzer Zeit zersetzt; in den meisten Fällen tritt diese Zersetzung innerhalb weniger Sekunden ein. Auf Grund dieser hohen Empfindlichkeit erfordert der Einsatz von Schrock-Alkylidenen des Molybdäns und Wolframs spezielle Arbeitstechniken sowie einen zum Teil erheblichen apparativen und präparativen Aufwand.

Schrock-Alkylidene des Molybdäns und Wolframs können durch Adduktbildung mit Donorliganden stabilisiert werden. Als Donorliganden wurden in der Literatur meist Ether und Phosphane eingesetzt, in einigen Fällen auch Stickstoffliganden wie Pyridin, Pyridinderivate oder Chinuclidin (Schrock, R. R., Crowe, W. E.; Bazan, G. C.; DiMare, M.; O'Regan, M. B.; Schofield, M. H. Organometallics 1991, 10, 1832); Ether-Addukte bleiben jedoch sehr luft- und feuchtigkeits-empfindlich. Die Stabilität der Phosphan-, Chinuclidin- oder Pyridinaddukte ist von der Donorstärke und der Größe der gewählten Phosphane, des Chinuclidins oder der gewählten Pyridine sowie von der Elektrophilie des gewählten Schrock-Alkylidenes abhängig. NMR Messungen zeigen, dass die Komplexbildung bereits bei Raumtemperatur in Lösung teilweise reversibel ist. In keinem Fall wurde über die Stabilität der gebildeten Addukte gegenüber Luft und/oder Feuchtigkeit berichtet.

Durch Komplexierung von Schrock Alkylidenen mit Hydrotris(pyrazolyl)borat oder Hydrotris(3,5-dimethylpyrazolyl)borat lassen sich hingegen luftstabile Addukte herstellen. Allerdings kann weder Hydrotris(pyrazolyl)borat noch Hydrotris(3,5-dimethylpyrazolyl)borat durch Zugabe von Lewis-Säuren wie zum Beispiel AlCl₃ von diesen Addukten abgelöst werden, sodass sich der ursprüngliche Schrock AlkylidenKomplex nicht wieder freisetzen lässt. Vielmehr führt die Zugabe von Lewis-Säuren wie zum Beispiel AlCl₃ zur Reaktion mit anderen Liganden des gebildeten Addukts und damit zur Bildung von einem oder mehreren neuen, strukturell nicht vollständig aufgeklärten Verbindungen (J. M. Boncella et al., J. Am. Chem. Soc. 1991, 113, 7066; Organometallics 1992, 11, 2342; Organometallics 1993, 12, 2814*;* J. Organomet. Chem. 1995, 485, 37).

In einem Fall wurde ein Bipyridin-Addukt eines Schrock-Methyliden-Komplexes 1 als solches beschrieben (Fox, H. H.; Lee, J.-K.; Park, L. Y.; Schrock, R. R. Organometallics 1993, 12, 759) bei dem die Alkylideneinheit terminal, d. h. nicht weiter substituiert ist. Eine weitere Umsetzung des Methyliden-Komplexes 1 wurde nicht beschrieben.

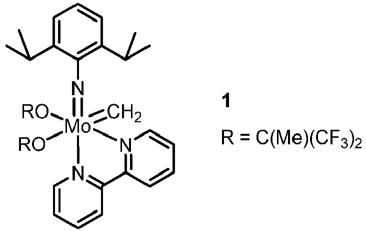

Überraschenderweise hat sich nun herausgestellt, dass sich Schrock-Alkylidene des Molybdäns und Wolframs mit nicht-terminaler Alkylideneinheit durch Komplexierung (Adduktbildung) mit bidentaten Stickstoffliganden, wie zum Beispiel 1,10-Phenathrolin oder 2,2'-Bipyridin (2,2'-Dipyridyl) bzw. deren Derivaten soweit stabilisieren lassen, dass sie ohne besondere Vorsichtsmaßnahmen an der Luft gehandhabt und über lange Zeit ohne besondere Vorkehrungen bei Raumtemperatur an Luft gelagert werden können, ohne dass Zersetzung eintritt. In einigen Fällen sind diese Addukte an der Luft bei Raumtemperatur über Monate lagerbar, ohne das Zersetzung eintritt. Allerdings weisen diese Komplexe selbst unter den bei Metathesereaktionen üblichen Reaktionsbedingungen *keine* nennenswerte katalytische Aktivität auf. Im Gegensatz zum bisherigen Stand des Wissens wurde jedoch überraschend gefunden, dass aus den genannten, aus Schrock-Alkylidenen des Molybdäns und Wolframs und bidentaten Stickstoffliganden hergestellten Komplexen (Addukten) mit Hilfe geeigneter Additiva in einem inerten Lösungsmittel die katalytisch aktiven Schrock-Alkylidene unverändert und in guten Ausbeuten wieder freigesetzt werden können; dadurch wird deren hohe katalytische Aktivität wiederhergestellt. Das in dieser Erfindung beschriebene Vorgehen zur Darstellung und Nutzung luftstabiler Addukte und der Freisetzung der aktiven Spezies durch Reaktion dieser Addukte mit geeigneten Additiva vereinfacht die Handhabung und Nutzung von Schrock-Alkylidene des Molybdäns und Wolframs erheblich, da weder spezielle Arbeitstechniken noch ein besonderer apparativer Aufwand nötig sind.

Gegenstand der vorliegenden Erfindung sind demnach Metallkomplexe der allgemeinen Formel I 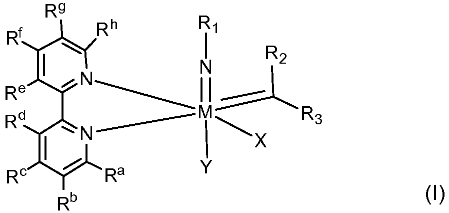 in der
M für Mo oder W steht,
R¹ für C₁-C₁₂ Alkyl, 5- bis 18-gliedriges Aryl, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können, die Substituenten X, Y gleich oder verschieden sein können und unabhängig voneinander ausgewählt werden können aus: Halogen, Methansulfonyloxy, Trifluormethansulfonyloxy, Benzolsulfonyloxy, Toluolsulfonyloxy, Pyrrolyl, C₁-C₁₂-Alkyloxy, 5- bis 18-gliedriges Aryloxy, Tri(C₁-C₁₂-Alkyl)silyloxy, Di(C₁-C₁₂-Alkyl)(C₆-C₁₈-Aryl)silyloxy, (C₁-C₁₂-Alkyl)di(C₆-C₁₈-Aryl)silyloxy, Tris(C₁-C₁₂-Alkyloxy)silyloxy, die ihrerseits mit einem oder mehreren gleichen oder verschiedenen C₁-C₁₂-Alkyl, 5- bis 18-gliedriges Aryl, C₁-C₁₂-Alkoxy, Di-(C₁-C₄-Alkyl)amino, Halogen, Trifluormethyl, Cyano, Nitro Resten substituiert sein können,
R² für H, C₁-C₁₂-Alkyl, 5- bis 18-gliedriges Aryl, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können, stehen,
R³ für C₁-C₁₂-Alkyl, 5- bis 18-gliedriges Aryl, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können, stehen,
R^{a}, R^{b}, R^{c}, R^{d}, R^{e}, R^{f}, R^{g}, R^{h} gleich oder verschieden sein können und unabhängig voneinander ausgewählt werden können aus H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können, oder die Reste R^{d} und R^{e} unter Bildung eines 5 bis 8 gliedrigen Ringes mit einander verbunden sind,

Die Reste R^{d} und R^{e} können auch unter Bildung eines 5 bis 8 gliedrigen Ringes mit einander verbunden sein und gemeinsam für einen Rest R⁴⁸ stehen, der CR⁴⁹R⁵⁰, CR⁵¹-CR⁵² CR⁵³R⁵⁴-CR⁵⁵R⁵⁶ CR⁵⁷R⁵⁸-CR⁵⁹R⁶⁰-CR⁶¹R⁶², CR⁶²R⁶³=CR⁶⁴R⁶⁵-CR⁶⁶R⁶⁷, CR⁶⁹R⁷⁰-CR⁷¹R⁷²-CR⁷³R⁷⁴⁻CR⁷⁵CR⁷⁶, CR⁷⁷=CR⁷⁸-CR⁷⁹R⁸⁰-CR⁸¹CR⁸² und/oder CR⁸³R⁸⁴-CR⁸⁵=CR⁸⁷-CR⁸⁸CR⁸⁹ bedeutet, in welchen R⁴⁹, R⁵⁰, R⁵¹, R⁵² R⁵³, R⁵⁴, R⁵⁵, R⁵⁶, R⁵⁷, R⁵⁸, R⁵⁹, R⁶⁰, R⁶¹, R⁶² R⁶³, R⁶⁴, R⁶⁵, R⁶⁶, R⁶⁷, R⁶⁸, R⁶⁹, R⁷⁰, R⁷¹, R⁷², R⁷³, R⁷⁴, R⁷⁵, R⁷⁶, R⁷⁷, R⁷⁸, R⁷⁹, R⁸⁰, R⁸¹, R⁸², R⁸³, R⁸⁴, R⁸⁵, R⁸⁶, R⁸⁷, R⁸⁸ und R⁸⁹ jeweils unabhängig von einander ausgewählt sind und die gleiche Bedeutung haben können wie R^{a}.

Die Metallkomplexe mit der Formel I bilden an Luft stabile Verbindungen. Es wird vermutet, dass die Stabilisierung durch Komplexierung an geeignete Stickstoffliganden erfolgt.

Bevorzugte Verbindungen der vorliegenden Erfindung sind durch folgende Formel 2 wiedergegeben, 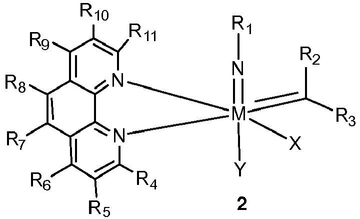 worin
M, R¹, R², R³, X und Y wie oben definiert sind,
die Substituenten R⁴, R⁵, R⁶, R⁷, R⁸, R⁹, R¹⁰, R¹¹ gleich oder verschieden sein können und unabhängig voneinander ausgewählt werden können aus: H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

Im Rahmen der vorliegenden Erfindung bedeuten:
"C₁-C₁₂-Alkyl" einen geradkettigen, verzweigten oder cyclischen Alkylrest mit 1 bis 12 Kohlenstoffatomen.
"5- bis 18-gliedriges Aryl" einen mono-, bi- oder tricyclischen carbocyclischen oder heterocyclischen aromatischen Rest, der seinerseits 0 bis 5 Substituenten tragen kann aus der Liste: C₁-C₁₂-Alkyl, C₁-C₁₂-Alkoxy, Di(C₁-C₄-Alkyl)amino, 5- bis 18-gliedriges Aryl, Halogen, Cyano, Nitro.
"Di-(C₁-C₄-Alkyl)amino" eine Amino-Gruppe mit zwei gleichen oder verschiedenen geradkettigen, verzweigten oder cyclischen Alkylsubstituenten, die jeweils 1 bis 4 Kohlenstoffatome aufweisen.
"Halogen" schließt im Rahmen der Erfindung Fluor, Chlor, Brom und Iod ein.

Bevorzugte Alkylreste im Rahmen dieser Erfindung sind geradkettige, verzweigte oder cyclische Alkylreste mit 1 bis 12 Kohlenstoffatomen. Beispielhaft und vorzugsweise seien genannt: Methyl, Trifluormethyl, Ethyl, n-Propyl, Isopropyl, Hexafluorisopropyl, n-Butyl, *iso*-Butyl, *sec*-Butyl, *tert*-Butyl, Trifluor-*tert*-butyl, Hexafluor-*tert*-butyl, Nonafluor-*tert*-butyl, 1-Ethylpropyl, n-Pentyl, n-Hexyl, Cyclopropyl, Cyclobutyl, Cyclopentyl, Cyclohexyl und Cycloheptyl, Adamantyl.

Bevorzugte Arylreste im Rahmen dieser Erfindung sind: Phenyl, Naphthyl, Anthryl, Methylphenyl, Dimethylphenyl, Trimethylphenyl, Ethylphenyl, Diethylphenyl, Isopropylphenyl, Di(isopropyl)phenyl, Tri(isopropyl)phenyl, tert-Butylphenyl, Di(tert-butyl)phenyl, Methoxyphenyl, Dimethoxyphenyl, Trimethoxyphenyl, Trifluormethylphenyl, Bis(trifluormethyl)phenyl, Fluorphenyl, Difluorphenyl, Chlorphenyl, Dichlorphenyl, Bromphenyl, lodphenyl, Pentafluorphenyl, Dimethylaminophenyl, Phenylphenyl, Diphenylphenyl, (Methoxycarbonyl)phenyl, (Ethoxycarbonyl)phenyl, (tert-Butoxycarbonyl)phenyl; ebenso bevorzugt sind heterocyclische Arylreste mit bis zu zwei Heteroatomen aus der Reihe N, O und/oder S wie beispielsweise Furyl, Thienyl, Thiazolyl, Oxazolyl, Indolyl, Isothiazolyl, Isoxazolyl, Pyrazolyl, Imidazolyl, Pyridyl, Pyrimidinyl, Pyridazinyl, Pyrazinyl, Benzofuranyl, Benzothiophenyl, Benzimidazolyl, Carbazolyl.

Bevorzugte Di-(C₁-C₄-Alkyl)amino Reste im Rahmen dieser Erfindung sind folgende Di-C₁-C₄-Alkylamino Reste: *N,N*-Dimethylamino, *N,N-*Diethylamino, *N*-Ethyl-*N*-methylamino, *N*-Methyl-*N*-n-propylamino, *N*-Isopropyl-*N*-n-propylamino, *N,N*-Diisopropylamino, *N*-n-Butyl-*N*-methylamino und *N*-*tert*-Butyl-*N*-methylamino.

Weiterhin bevorzugte Verbindungen im Rahmen der vorliegenden Erfindung sind Komplexe der allgemeinen Formel **3** 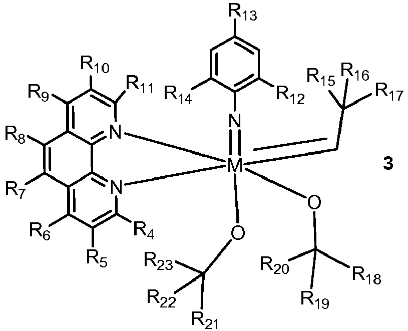 worin
M = Mo, W,
die Substituenten R¹², R¹³, R¹⁴ gleich oder verschieden sein können und unabhängig voneinander ausgewählt werden können aus: H, Halogen, Trifluormethyl, Methyl, Ethyl, Propyl, Butyl, iso-Propyl, tert-Butyl,
die Substituenten R¹⁵, R¹⁶, R¹⁷ gleich oder verschieden sein können und unabhängig voneinander ausgewählt werden können aus: H, Methyl, Ethyl, Propyl, Phenyl,
die Substituenten R¹⁸, R¹⁹, R²⁰, R²¹, R²², R²³ gleich oder verschieden sein können und unabhängig voneinander ausgewählt werden können aus: H, Methyl, Trifluormethyl,
die Substituenten R⁴, R⁵, R⁶, _{R}⁷, R⁸, R⁹, R¹⁰, R¹¹ gleich oder verschieden sein können und unabhängig voneinander ausgewählt werden können aus: H, C₁-C₆-Alkyl, C₁-C₆-Alkyloxy, C₁-C₆-Alkyloxycarbonyl, Phenyl, Halogen, Nitro.

Ebenfalls bevorzugte Verbindungen im Rahmen der vorliegenden Erfindung sind Komplexe der allgemeinen Formel **4** 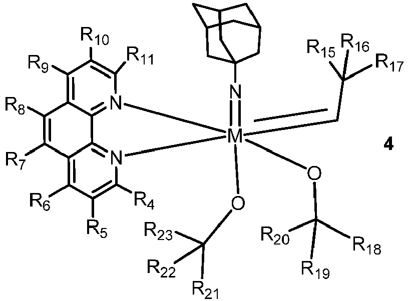 worin
M = Mo,
die Substituenten R¹⁵, R¹⁶, R¹⁷ gleich oder verschieden sein können und unabhängig voneinander ausgewählt werden können aus: H, Methyl, Ethyl, Propyl, Phenyl,
die Substituenten R¹⁸, R¹⁹, R²⁰, R²¹, R²², R²³ gleich oder verschieden sein können und unabhängig voneinander ausgewählt werden können aus: H, Methyl, Trifluormethyl,
die Substituenten R⁴, R⁵, R⁶, R⁷, R⁸, R⁹, R¹⁰, R¹¹ gleich oder verschieden sein können und unabhängig voneinander ausgewählt werden können aus: H, C₁-C₆-Alkyl, C₁-C₆-Alkyloxy, C₁-C₆-Alkyloxycarbonyl, Phenyl, Halogen, Nitro.

Beispiele für bevorzugte 1,10-Phenanthroline, die im Rahmen dieser Erfindung als Liganden zur Stabilisierung von Schrock-Alkyliden Komplexen des Molybdäns oder Wolframs eingesetzt werden können, sind: 1,10-Phenanthrolin, 4-Methyl-1,10-phenanthrolin, 5-Methyl-1,10-phenanthrolin, 2,9-Dimethyl[1,10]phenanthrolin, 5,6-Dimethyl-1,10-phenanthrolin, 5-Chlor[1,10]phenanthrolin, 4,7-Dichloro-1,10-phenanthrolin, 4,7-Dichlor-1,10-phenanthrolin, 3,4,7,8-Tetramethyl-1,10-phenanthrolin, 4,7-Diphenyl[1,10]phenanthrolin, 2,9-Dimethyl-4,7-diphenyl[1,10]phenanthrolin, 5-Nitro-1,10-phenanthrolin, 4,7-Dimethoxy-1,10-phenanthrolin.

Besonders bevorzugte Verbindungen im Rahmen der vorliegenden Erfindung sind Komplexe der allgemeinen Formel **5-8** 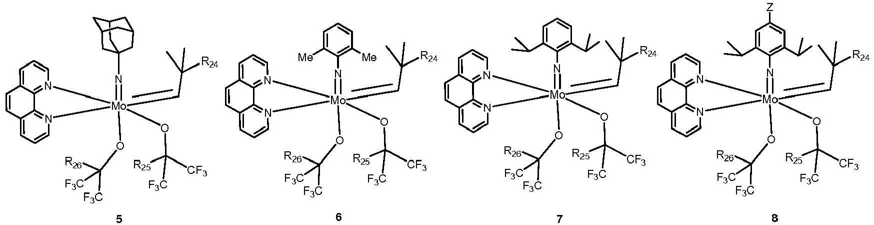 worin
R²⁴ = Me, Ph
R²⁵, R²⁶ = H, Me, CF₃
Z = Methyl, iso-Propyl, Halogen

Ebenso sind Gegenstand der vorliegenden Erfindung Komplexe der allgemeinen Formel 9, Methoden zu deren Herstellung, sowie ihre Anwendung als Präkatalysatoren für die Alkenmetathese, 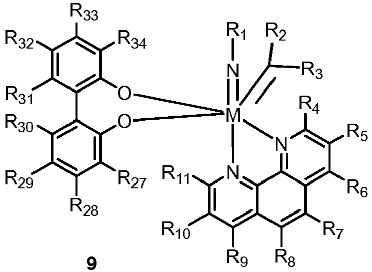 worin
M, R¹, R², R³ und R⁴, R⁵, R⁶, R⁷, R⁸, R⁹, R¹⁰, R¹¹ wie oben definiert sind,
die Substitutenten R²⁷, R²⁸, R²⁹, R³⁰, R³¹, R³², R³³, R³⁴ gleich oder verschieden sein können und unabhängig voneinander ausgewählt werden können aus: H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können,

Ebenso sind Gegenstand der vorliegenden Erfindung Komplexe der allgemeinen Formel 10, Methoden zu deren Herstellung, sowie ihre Anwendung als Präkatalysatoren für die Alkenmetathese, 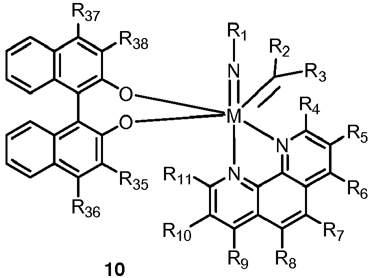 worin
M, die Substituenten R¹, R², R³, R⁴, R⁵, R⁶, R⁷, R⁸, R⁹, R¹⁰ und R¹¹ wie oben definiert sind und
die Substituenten R³⁵, R³⁶ , R³⁷ , R³⁸ gleich oder verschieden sein können und unabhängig voneinander ausgewählt werden können aus: H, Halogen, Nitro, Cyano, Trifluormethyl, C₁-C₁₂-Alkyl, 5- bis 18-gliedriges Aryl, C₁-C₁₂-Alkyloxy, Di-(C₁-C₄-Alkyl)amino, Tri(C₁-C₁₂-Alkyl)silyl, Di(C₁-C₁₂-Alkyl)(C₆-C₁₈-Aryl)silyl, (C₁-C₁₂-Alkyl)di(C₆-C₁₈-Aryl)silyl, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

Beispiele für besonders bevorzugte Verbindungen im Rahmen der vorliegenden Erfindung sind Komplexe der allgemeinen Formeln **11-16,** 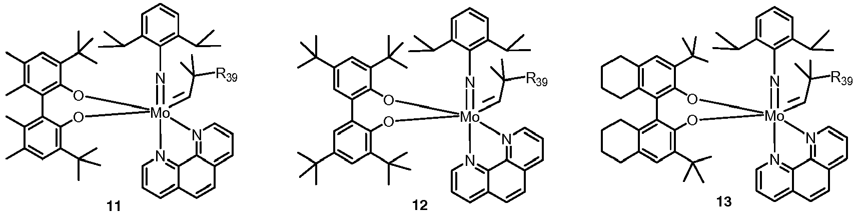 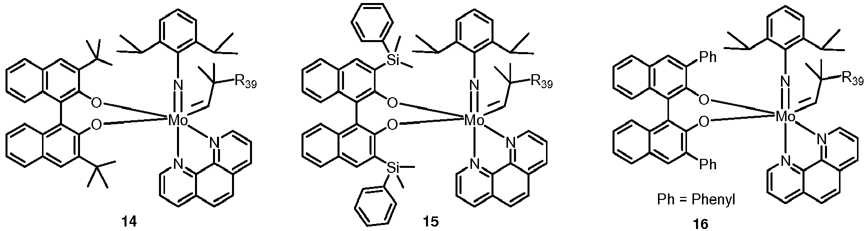 worin
R³⁹ = Me, Phenyl.

Ebenso sind Gegenstand der vorliegenden Erfindung Komplexe der allgemeinen Formel **17,** Methoden zu deren Herstellung, sowie ihre Anwendung als Präkatalysatoren für die Alkenmetathese, 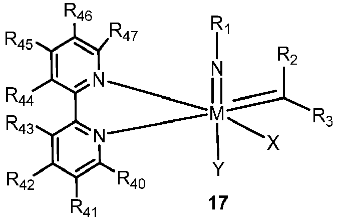 worin
M = Mo, W,
der Substituent R¹ ausgewählt werden kann aus: C₁-C₁₂-Alkyl, 5- bis 18-gliedriges Aryl, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können,
die Substituenten X, Y gleich oder verschieden sein können und unabhängig voneinander ausgewählt werden können aus: Halogen, Methansulfonyloxy, Trifluormethansulfonyloxy, Benzolsulfonyloxy, Pyrrolyl, C₁-C₁₂-Alkyloxy, 5- bis 18-gliedriges Aryloxy, Tri(C₁-C₁₂-Alkyl)silyloxy, Di(C₁-C₁₂-Alkyl)(C₆-C₁₈-Aryl)silyloxy, (C₁-C₁₂-Alkyl)di(C₆-C₁₈-Aryl)silyloxy, Tris(C₁-C₁₂-Alkyloxy)silyloxy, die ihrerseits mit einem oder mehreren gleichen oder verschiedenen C₁-C₁₂-Alkyl, 5- bis 18-gliedriges Aryl, C₁-C₁₂-Alkoxy, Di-(C₁-C₄-Alkyl)amino, Halogen, Trifluormethyl, Cyano, Nitro Resten substituiert sein können,
der Substituent R₂ ausgewählt werden kann aus den Resten: H, C₁-C₁₂-Alkyl, 5- bis 18-gliedriges Aryl, wobei jeder dieser Reste seinerseits mit einem oder mehreren gleichen oder verschiedenen C₁-C₁₂-Alkyl, 5- bis 18-gliedriges Aryl, C₁-C₁₂ Alkyloxy, Di-(C₁-C₄-Alkyl)amino, Halogen, Trifluormethyl, Cyano, Nitro Resten substituiert sein kann,
der Substituent R³ ausgewählt werden kann aus den Resten: C₁-C₁₂-Alkyl, 5- bis 18-gliedriges Aryl, wobei jeder dieser Reste seinerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein kann,
die Substituenten R⁴⁰, R⁴¹, R⁴², R⁴³, R⁴⁴, R⁴⁵, R⁴⁶, R⁴⁷ gleich oder verschieden sein können und unabhängig voneinander ausgewählt werden können aus: H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂ Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

Bevorzugte Verbindungen im Rahmen der vorliegenden Erfindung sind Komplexe der allgemeinen Formel **18** 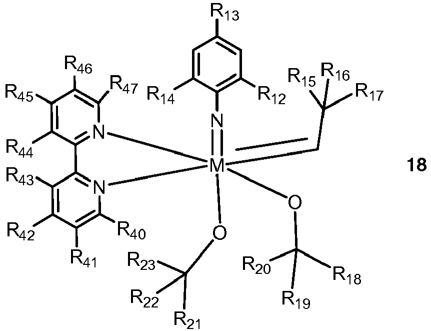 worin
M = Mo, W,
die Substituenten R¹², R¹³, R¹⁴ gleich oder verschieden sein können und unabhängig voneinander ausgewählt werden können aus: H, Halogen, Trifluormethyl, Methyl, Ethyl, Propyl, Butyl, iso-Propyl, tert-Butyl,
die Substituenten R¹⁵, R¹⁶ , R¹⁷ gleich oder verschieden sein können und unabhängig voneinander ausgewählt werden können aus: H, Methyl, Ethyl, Propyl, Phenyl
die Substituenten R¹⁸, R¹⁹, R²⁰, R²¹, R²², R²³ gleich oder verschieden sein können und unabhängig voneinander ausgewählt werden können aus: H, Methyl, Trifluormethyl,
die Substituenten R⁴⁰, R⁴¹, R⁴², R⁴³, R⁴⁴, R⁴⁵, R⁴⁶, R⁴⁷ gleich oder verschieden sein können und unabhängig voneinander ausgewählt werden können aus: H, C₁-C₆-Alkyl, C₁-C₆ Alkyloxy, C₁-C₆-Alkyloxycarbonyl, Phenyl, Halogen, Nitro.

Ebenfalls bevorzugte Verbindungen im Rahmen der vorliegenden Erfindung sind Komplexe der allgemeinen Formel **19** 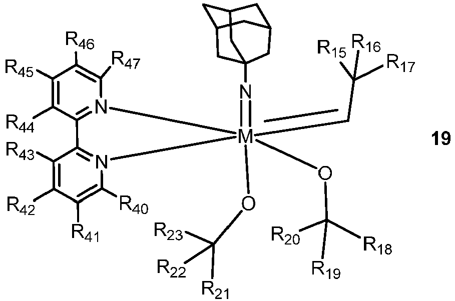 worin
M = Mo,
die Substituenten R¹⁵, R¹⁶, R¹⁷ gleich oder verschieden sein können und unabhängig voneinander ausgewählt werden können aus: H, Methyl, Ethyl, Propyl, Phenyl
die Substituenten R¹⁸, R¹⁹, R²⁰, R²¹, R²², R²³ gleich oder verschieden sein können und unabhängig voneinander ausgewählt werden können aus: H, Methyl, Trifluormethyl,
die Substituenten R⁴⁰, R⁴¹, R⁴², R⁴³, R⁴⁴, R⁴⁵, R⁴⁶, R⁴⁷ gleich oder verschieden sein können und unabhängig voneinander ausgewählt werden können aus: H, C₁-C₆-Alkyl, C₁-C₆-Alkyloxy, C₁-C₆-Alkyloxycarbonyl, Phenyl, Halogen, Nitro.

Beispiele für bevorzugte 2,2'-Bipyridine (2,2'-Dipyridyle), die im Rahmen dieser Erfindung als Liganden zur Stabilisierung von Schrock-Alkyliden Komplexen des Molybdäns oder Wolframs eingesetzt werden können, sind: 2,2'-Bipyridin, 5,5'-Dimethyl-2,2'-dipyridyl, 4,4'-Dimethyl-2,2'-dipyridyl, 6,6'-Dimethyl-2,2'-dipyridyl, 4-4'-Dimethoxy-2-2'-bipyridin, 2,2'-Bichinolin, 4,4'-Di-tert-butyl-2,2'-dipyridyl, 2,2'-Bipyridinyl-4,4'-dicarbonsäuredimethylester, 4,4'-Diphenyl-2,2'-dipyridyl, 6,6'-Dibromo-2,2'-dipyridyl, 4,4'-Dinonyl-2,2'-dipyridyl, 2,2'-Bichinolinyl-4,4'-dicarbonsäuredibutylester, 2,2'-Bichinolinyl-4,4'-dicarbonsäurediheptylester, 6-Methyl-2,2'-dipyridyl, 2-(2-Pyridinyl)chinolin, 2-Pyridin-2-yl-4-pyrrolidin-1-yl-chinolin, 4-Piperidin-1-yl-2-pyridin-2-yl-chinolin, 4-Morpholin-4-yl-2-pyridin-2-yl-chinolin.

Besonders bevorzugte Verbindungen im Rahmen der vorliegenden Erfindung sind Komplexe der allgemeinen Formel **20-23** 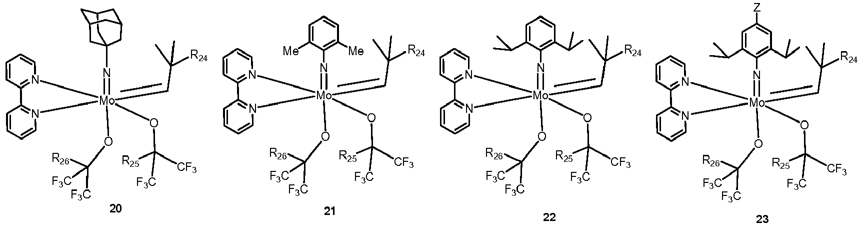 worin
R²⁴ = Me, Ph
R²⁵, R²⁶ = H, Me, CF₃
Z = Methyl, iso-Propyl, Halogen.

Ebenso sind Gegenstand der vorliegenden Erfindung Komplexe der allgemeinen Formel **24,** Methoden zu deren Herstellung, sowie ihre Anwendung als Präkatalysatoren für die Alkenmetathese, 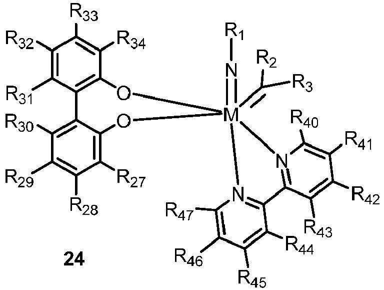 worin
M, R¹, R² und R³ sowie R²⁷, R²⁸, R²⁹, R³⁰, R³¹, R³², R³³, R³⁴, R⁴⁰, R⁴¹, R⁴², R⁴³, R⁴⁴, R⁴⁵, R⁴⁶ und R⁴⁷ wie oben definiert sind.

Ebenso sind Gegenstand der vorliegenden Erfindung Komplexe der allgemeinen Formel **25,** Methoden zu deren Herstellung, sowie ihre Anwendung als Präkatalysatoren für die Alkenmetathese, 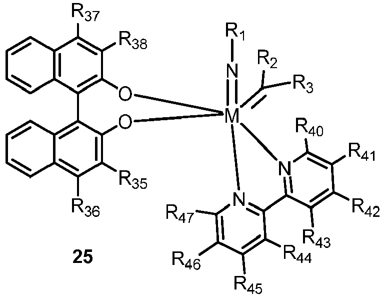 worin
M, R¹, R² und R³ sowie R³⁵, R³⁶, R³⁷, R³⁸, R⁴⁰, R⁴¹, R⁴², R⁴³, R⁴⁴, R⁴⁵, R⁴⁶ und R⁴⁷ wie oben definiert sind.

Beispiele für besonders bevorzugte Verbindungen im Rahmen der vorliegenden Erfindung sind Komplexe der allgemeinen Formeln **26-31** 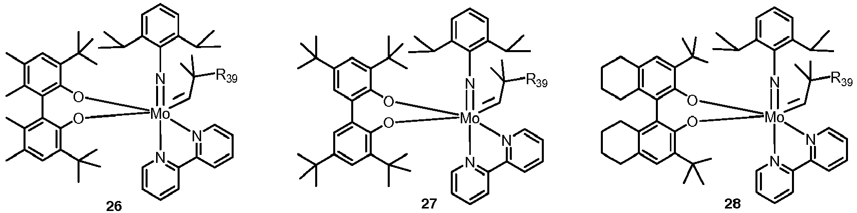 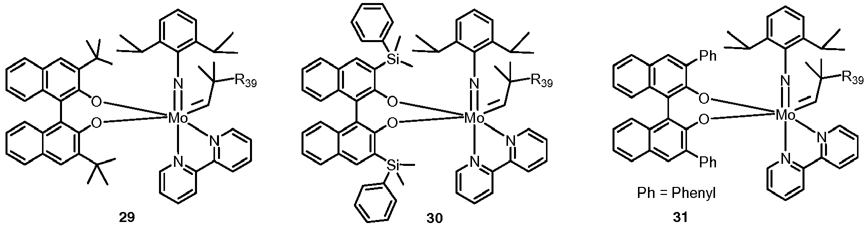 worin
R³⁹ = Me, Phenyl.

Ein weiterer Gegenstand der vorliegenden Erfindung ist ein Verfahren zur Herstellung der Verbindungen der allgemeinen Formeln **I** 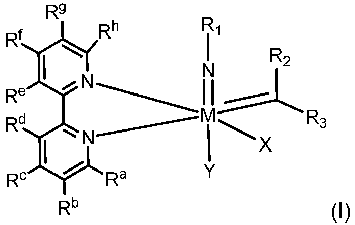 worin
M, X, Y, R¹, R², R³, R^{a}, R^{b}, R^{c}, R^{d}, R^{e}, R^{f}, R^{g}, R^{h} wie oben definiert sind,
in welchem eine Verbindung der Formel 32 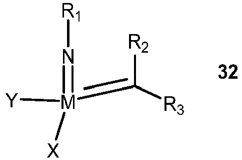 worin
M, X, Y, R₁, R₂, R₃ wie oben definiert sind,
mit einer Verbindung der allgemeinen Formel **II** 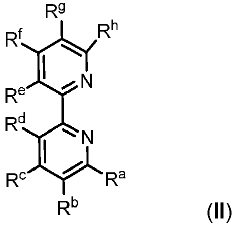 worin
R^{a}, R^{b}, R^{c}, R^{d}, R^{e}, R^{f}, R^{g}, R^{h} wie oben definiert sind,
umgesetzt wird.

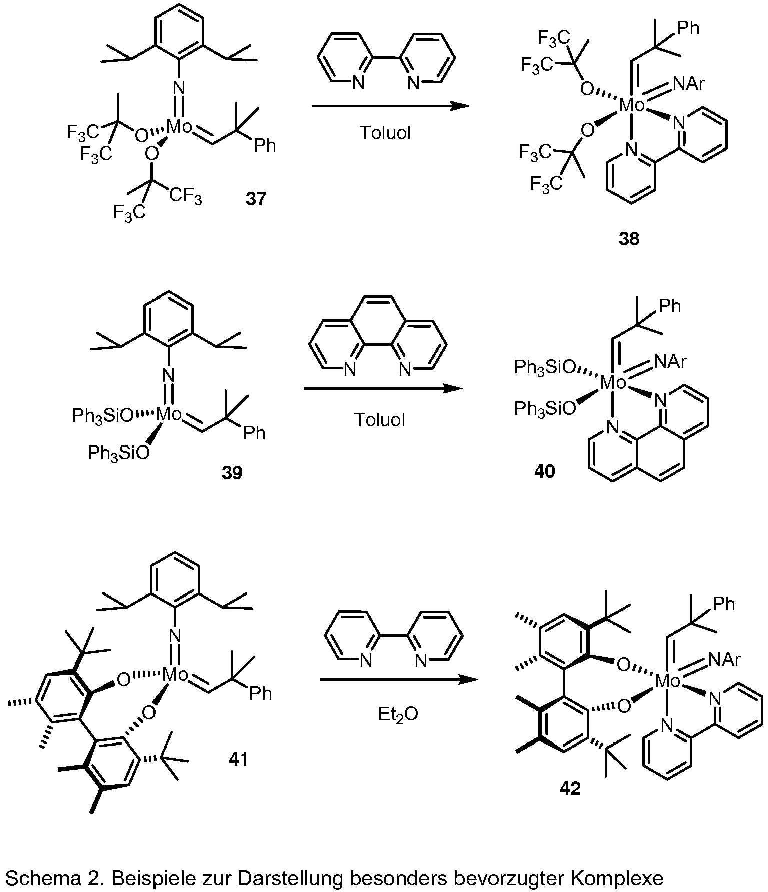

Beispiele für die Herstellung besonders bevorzugter Komplexe nach dem genannten Verfahren sind in Schema 2 gezeigt und werden im Abschnitt "Beispiele" im Detail beschrieben.

Ebenfalls Gegenstand der vorliegenden Erfindung ist ein Verfahren zur Nutzung der Komplexe der Formeln **I, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 38, 40, 42, 43** als Präkatalysatoren zur Durchführung von Alkenmetathese Reaktionen durch Aktivierung mit einem geeigneten Additiv. Bei diesem Verfahren wird eine Lösung oder eine Suspension von Verbindung der Formeln **I, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 38, 40, 42, 43** in einem geeigneten Lösungsmittel mit wasserfreien Metallsalzen oder Metallkomplexen als Additiva umgesetzt, die ihrerseits stabile 1,10-Phenanthrolinkomplexe beziehungsweise 2,2'-Bipyridinkomplexe bilden. Diese Aktivierung erfolgt durch Dekomplexierung des jeweiligen 1,10-Phenanthrolin beziehungsweise 2,2'-Bipyridin Liganden vom Molybdän oder Wolfram unter Bildung der entsprechenden 1,10-Phenanthrolinkomplexe beziehungsweise 2,2'-Bipyridinkomplexe der verwendeten Additiva, welche wahlweise im Reaktionsgemisch verbleiben oder vor der Alkenmetathese aus dem Gemisch abgetrennt werden können. Für diese Aktivierung kommen alle wasserfreien Metallsalze oder Metallkomplexe in Betracht, die stabile 1,10-Phenanthrolinkomplexe beziehungsweise 2,2'-Bipyridinkomplexe bilden. Besonders bevorzugte Beispiele sind in Schema 3 sowie im Abschnitt "Beispiele" genannt.

Zu den bevorzugten Additiva zählen: AlX₃, MnX₂, FeX₂, FeX₃, CoX₂, CuX₂, ZnX₂, MgX₂, NiX₂, PdX₂, PtX₂, RuX₂, RuX₃, EuX₃, mit X = F, Cl, Br, I, acetylacetonat, sulfat, sulfonat, nitrat, acetat, trifluoracetat, trifluormethansulfonat (triflat).

Besonders bevorzugte Additiva sind: MgCl₂, MgBr₂, Mgl₂, MnCl₂, MnBr₂, MnI₂, FeCl₃, AlCl₃, CuCl₂, ZnCl₂, ZnBr₂, ZnI₂, Zn(triflat)₂, Zn(trifluoracetat)₂.

Abhängig vom gewählten Additiv und Addukt kann die Aktivierung bei unterschiedlichen Temperaturen durchgeführt werden, bevorzugt bei Temperaturen zwischen -20°C und +140°C, besonders bevorzugt bei Temperaturen zwischen 20°C und 110°C. Als Lösungsmittel kommen alle Lösungsmittel in Betracht, die nicht zur Zersetzung der freigesetzten Schrock-Alkyliden Komplexen des Molybdäns oder Wolframs führen; bevorzugte Lösungsmittel sind Kohlenwasserstoffe, Halogenkohlenwasserstoffe und Ether; besonders bevorzugt sind Pentane, Hexane, Heptane, Octane, Petrolether, Benzol, Toluol, Xylole, Cumol, Decalin, Chlorbenzol, Brombenzol, Fluorbenzol, Trifluormethylbenzol, Dichlorbenzole, Trichlorbenzole, Dichlormethan, Chloroform, Tetrachlorkohlenstoff, 1,2-Dichlorethan, Tetrahydrofuran, 1,4-Dioxan, Dimethoxyethan, Diethylether, tert-Butylmethylether. Auch Mischungen dieser Lösungsmittel können eingesetzt werden.

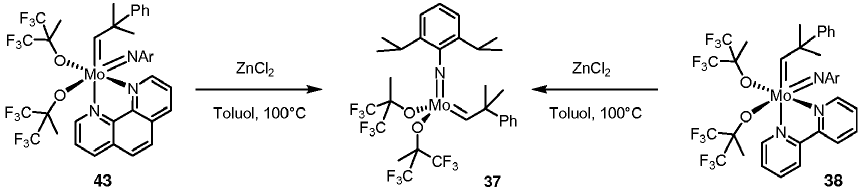

Schema 3. Beispiel der Aktivierung eines katalytisch inaktiven Addukts aus einem Schrock-Alkyliden und 1,10-Phenanthrolin (**43**) bzw. 2,2'-Bipyridin (**38**) unter Verwendung von ZnCl₂ als besonders bevorzugtem Additiv in Toluol als einem besonders bevorzugten Lösungsmittel gemäß der vorliegenden Erfindung. Erfolgt die Umsetzung in deuteriertem Toluol, so kann die Freisetzung des in der Alkenmetathese katalytisch aktiven Schrock-Alkylidens **37** durch ¹H NMR Spektroskopie verfolgt werden.

### Beispiele

Abkürzung: phen = 1,10-Phenanthrolin; bipy = 2,2'-Bipyridin; dme = 1,2-Dimethoxyethan; biphen = biphenyl-2,2'-diolat; TMS = Trimethylsilyl; TBS = tert-Butyldimethylsilyl; TBDPS = tert-Butyldiphenylsilyl.

Die zur Darstellung der Komplexe verwendeten 2,2'-Bipyridin Derivate wurden in Toluol gelöst und die Lösung über Molsieb 5Å (MS 5Å) getrocknet. Anschließend wurde das Molsieb abfiltriert, das Filtrat eingeengt und das verbleibende 2,2'-Bipyridin Derivat unter Argon aufbewahrt.

Die zur Darstellung der Komplexe verwendeten 1,10-Phenanthrolin Derivate wurden entweder durch doppelte Sublimation im Hochvakuum gereinigt und getrocknet, oder in Toluol gelöst und die Lösung über Molsieb 5Å (MS 5Å) getrocknet; anschließend wurde das Toluol im Vakuum abdestilliert und das verbleibende 1,10-Phenanthrolin Derivat unter Argon aufbewahrt.

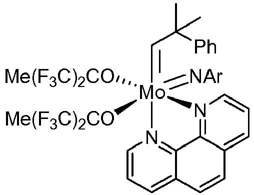

### [Mo(CHCMe₂Ph)(NAr)(OCMe(CF₃)₂)₂(phen)] (Ar = 2,6-diisopropylphenyl).

Eine Lösung von [Mo(CHCMe₂Ph)(NAr)(OCMe(CF₃)₂)₂] (326 mg, 426 µmol) in Toluol (3 mL) wurde zu einer Lösung von 1,10-Phenanthrolin (77 mg, 426 µmol) in Toluol (2 mL) zugegeben und das Gemisch 1 h bei Raumtemperatur gerührt. Anschließend wurde der gebildete Niederschlag abfiltriert und mit kaltem Toluol (ca. 2 mL) gewaschen. Die vereinten Filtrate wurden auf etwa ein Drittel des ursprünglichen Volumens eingeengt und anschließend auf -40°C abgekühlt, was eine zweite Charge an Komplex ergab. Die Chargen wurden vereinigt und im Vakuum getrocknet. Gelber Feststoff (384 mg, 95%). ¹H NMR (400 MHz, CD₂Cl₂): δ = 13.88 (s, 1H), 9.48 (dd, *J =* 4.7, 1.6 Hz, 1H), 8.41 (dd, *J =* 8.3, 1.6 Hz, 1 H), 8.37 (dd, *J* = 8.3, 1.6 Hz, 1 H), 7.98 (dd, *J* = 5.2, 1.3 Hz, 1 H), 7.92 (d, *J* = 8.9 Hz, 1H), 7.86-7.82 (m, 2H), 7.67 (dd, *J =* 8.5, 1.0 Hz, 2H), 7.46 (t, *J =* 8.1 Hz, 2H), 7.33 (t, *J =* 7.3 Hz, 1 H), 7.30 (dd, *J =* 8.2, 5.6 Hz, 1 H), 6.91 (br s, 1 H), 6.80 (t, *J =* 7.6 Hz, 1H), 6.71 (br s, 1H), 4.38 (br s, 1H), 3.13 (br s, 1H), 2.07 (s, 3H), 1.68 (s, 3H), 1.65 (s, 3H), 1.19 (br s, 6H), 0.85 (br s, 3H), 0.26 (s, 3H), -0.55 ppm (br s, 3H); ¹³C NMR (100 MHz, CD₂Cl₂): δ = 309.6, 158.9, 150.5, 150.0, 149.6, 149.5, 145.8, 143.4, 139.3, 138.4, 138.0, 130.4, 129.9, 129.4, 128.7, 128.6, 127.9, 126.9, 126.7, 126.4, 126.3, 125.6, 125.1, 124.9, 80.1, 55.0, 32.0, 28.8, 28.2, 26.9, 26.6, 24.7, 24.3, 22.6, 21.5, 17.9, 17.6 ppm; ¹⁹F NMR (377 MHz, CD₂Cl₂): δ = -76.3 (q, *J* = 10.3 Hz, 3F), -77.4 (m, 3F), -77.6 (q, *J* = 10.3 Hz, 3F), -77.9 ppm (m, 3F); IR (Film, cm⁻¹): 2966, 2868, 1578, 1424, 1296, 1214, 1163, 1105, 1070, 958, 844, 762, 727, 697.

Laut ¹H NMR ist dieser Komplex nach vierwöchiger Lagerung bei Raumtemperatur an Luft zu ca. 50% intakt.

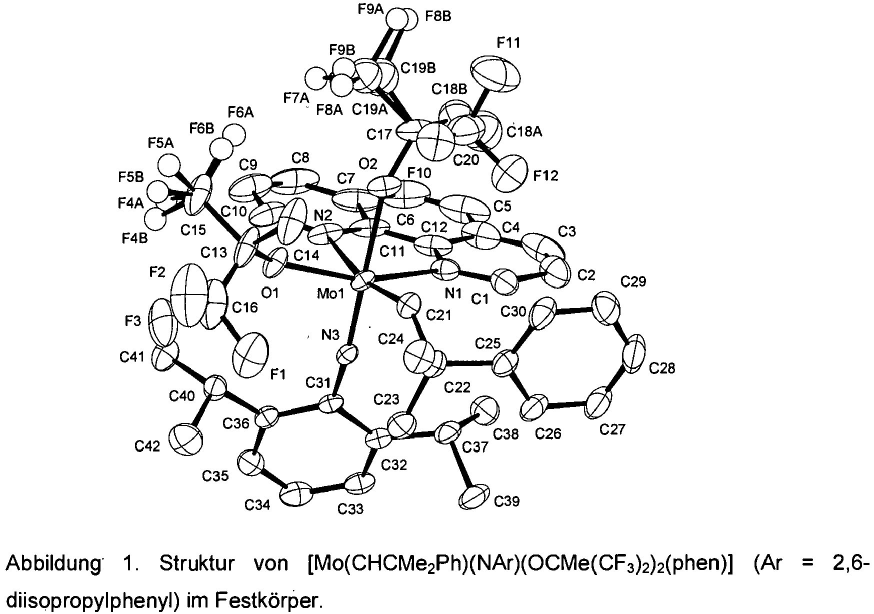

### [Mo(CHCMe₂Ph)(NAr)(OCMe(CF₃)₂)₂(bipy)] (Ar = 2,6-diisopropylphenyl).

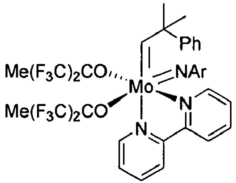

Eine Lösung von [Mo(CHCMe₂Ph)(NAr)(OCMe(CF₃)₂)₂] (360 mg, 470 µmol) in Toluol (3 mL) wurde zu einer Lösung von 2,2'-Bipyridin (73 mg, 470 µmol) in Toluol (2 mL) zugegeben und das Gemisch 1 h bei Raumtemperatur gerührt. Anschließend wurde der gebildete Niederschlag abfiltriert und mit kaltem Toluol (ca. 2 mL) gewaschen. Die vereinten Filtrate wurden auf etwa ein Drittel des ursprünglichen Volumens eingeengt und anschließend auf -40°C abgekühlt, was eine zweite Charge an Komplex ergab. Die Charge wurden vereinigt und im Vakuum getrocknet. Gelber Feststoff (382 mg, 88%). ¹H NMR (400 MHz, CD₂Cl₂): δ = 13.74 (s, 1 H), 9.22 (dd, *J* = 5.1, 1.6 Hz, 1 H), 8.02 (d, *J =* 8.0 Hz, 1H), 7.97 (d, *J* = 8.1 Hz, 1H), 7.92-7.84 (m, 2H), 7.76 (d, *J* = 4.6 Hz, 1H), 7.62 (d, *J =* 8.2 Hz, 2H), 7.54 (ddd, *J* = 7.4, 5.2, 1.3 Hz, 1 H), 7.41 (t, *J =* 7.4 Hz, 2H), 7.28 (tt, *J =* 7.3, 1.2 Hz, 1 H), 6.96 (ddd, *J* = 7.4, 5.6, 1.3 Hz, 1 H), 9.92 (br s, 1 H), 6.88 (t, *J* = 7.0 Hz, 1 H), 6.84 (br s, 1H), 4.19 (br s, 1H), 3.16 (br s, 1H), 2.02 (s, 3H), 1.63 (s, 3H), 1.61 (s, 3H), 1.24 (br s, 3H), 1.12 (br s, 3H), 0.81 (br s, 3H), 0.55 (s, 3H), -0.09 ppm (br s, 3H); ¹³C NMR (100 MHz, CD₂Cl₂): δ = 309.0, 159.7, 154.9, 153.1, 151.5, 150.3, 149.9, 149.7, 149.6, 148.0, 140.0, 139.2, 128.5, 126.8, 126.4, 126.3, 126.2, 122.8, 121.4, 80.1, 54.8, 31.9, 28.7, 28.5, 26.9, 26.1, 25.0, 24.5, 22.6, 17.7 ppm; ¹⁹F NMR (377 MHz, CD₂Cl₂): δ = -76.4 (q, *J* = 10.3 Hz, 3F), -77.1 (q, *J* = 10.3 Hz, 3F), -77.4 (m, 3F), -77.7 ppm (m, 3F); IR (Film, cm⁻¹): 2968, 2868, 1599, 1443, 1296, 1210, 1165, 1078, 1024, 955, 757, 697.

Laut ¹H NMR ist dieser Komplex nach vierwöchiger Lagerung bei Raumtemperatur an Luft intakt und zeigt keine (< 5%) erkennbare Zersetzung.

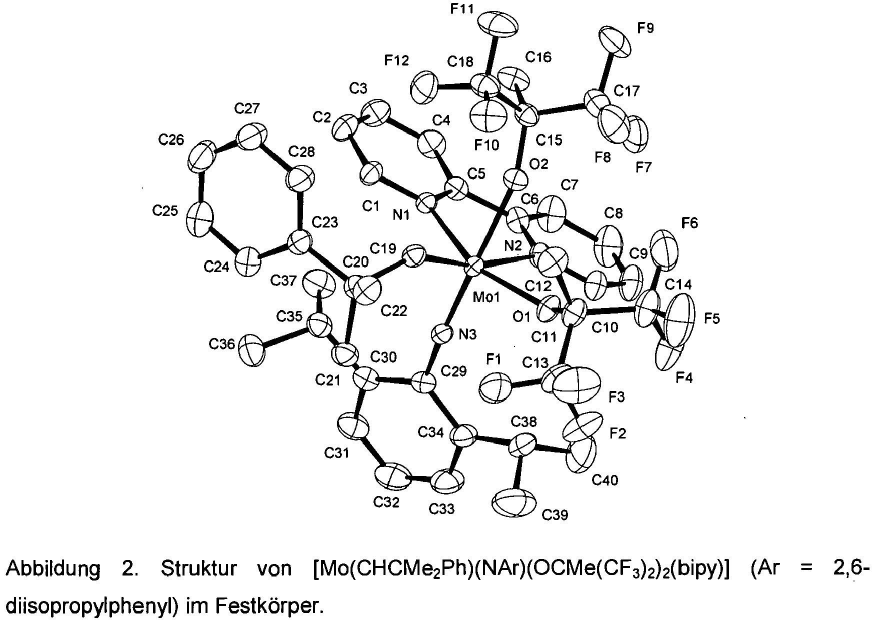

### [Mo(CH-tBu)(NAr)(OSiPh₃)₂(phen)] (Ar = 2,6-diisopropylphenyl).

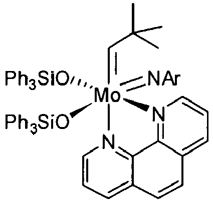

Eine Lösung von Ph₃SiOK (302 mg, 959 µmol) in Toluol (5 mL) wurde zu einer Lösung von [Mo(CH-*t*Bu)(NAr)(OSO₂CF₃)₂(dme)] (350 mg, 480 µmol) in Toluol (5 mL) zugegeben und das Gemisch 1 h bei Raumtemperatur gerührt. Anschließend wurde der gebildete Niederschlag abfiltriert und das Filtrat zu einer Lösung von 1,10 - Phenanthrolin (87 mg, 480 µmol) in Toluol (5 mL) gegeben. Das Gemisch wurde 1 h gerührt, anschließend das Lösungsmittel abgezogen, der Rückstand in wenig CH₂Cl₂ (≈ 5 mL) gelöst und das Produkt durch Zugabe von Pentan (25 mL) gefällt. Der Niederschlag wurde abfiltriert und im Vakuum getrocknet. Gelber Feststoff (417 mg, 81%). ¹H NMR (400 MHz, CD₂Cl₂): δ = 13.63 (s, 1H), 9.76 (dd, *J* = 4.7, 1.6 Hz, 1H), 9.30 (dd, *J* = 5.1, 1.5 Hz, 1 H), 8.26 (dd, *J =* 8.2, 1.6 Hz, 1 H), 7.94 (dd, J = 8.2, 1.4 Hz, 1 H), 7.84 (dd, *J* = 8.0, 1.4 Hz, 6H), 7.72 (d *J =* 8.8 Hz, 1 H), 7.61 (dd, *J* = 8.2, 4.7 Hz, 1 H), 7.50 (d, *J* = 8.8 Hz, 1 H), 7.41 (dd, *J* = 8.2, 5.2 Hz, 1 H), 7.23 (tt, *J* = 7.4, 1.4 Hz, 3H), 7.08-7.03 (m, 9H), 7.00 (dd, *J* = 8.0, 1.4 Hz, 6H), 6.80 (t, *J* = 7.4 Hz, 6H), 6.72-6.71 (m, 3H), 4.37 (br s, 1H), 3.66 (br s, 1H), 1.20 (s, 9H), 1.14 (br s, 3H), 0.50 (br s, 6H), -043 ppm (br s, 3H ); ¹³C NMR (100 MHz, CD₂Cl₂): δ = 304.9, 158.1, 150.2, 149.5, 145.0, 143.3, 141.4, 140.6, 138.6, 137.4, 136.7, 135.5, 135.3, 130.5, 130.1, 130.0, 128.5, 128.3, 128.0, 127.5, 127.4, 127.2, 127.0, 125.2, 124.9, 124.2, 123.1, 49.0, 32.0 ppm; IR (Film, cm⁻¹): 2936, 2866, 1513, 1423, 1256, 1144, 1106, 1032, 947, 842, 760, 698.

### [Mo(CHCMe₂Ph)(NAr)(OSiPh₃)₂(phen)] (Ar = 2,6-diisopropylphenyl).

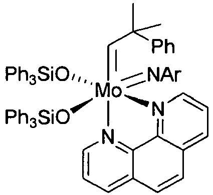

Hergestellt wie oben beschrieben aus Ph₃SiOK (300 mg, 955 µmol), [Mo(CHCMe₂Ph)(NAr)(OSO₂CF₃)₂(dme)] (378 mg, 477 µmol) und 1,10 - Phenanthrolin (86 mg, 477 µmol). Gelber Feststoff (460 mg, 85%). ¹H NMR (400 MHz, CD₂Cl₂): δ = 13.61 (s, 1 H), 9.78 (dd, *J* = 4.7, 1.6 Hz, 1 H), 8.22 (dd, *J* = 8.2, 1.5 Hz, 1 H), 7.91 (dd, *J =* 5.2, 1.5 Hz, 1 H), 7.85 (dd, *J* = 8.0, 1.4 Hz, 6H), 7.76 (dd, *J* = 8.2, 1.5 Hz, 1 H), 7.62-7.58 (m, 4H), 7.48 (t, *J* = 7.6 Hz, 2H), 7.36 (t, *J* = 8.8 Hz, 1 H), 7.25 (tt, *J* = 7.4, 1.4 Hz, 3H), 7.09-7.02 (m, 10H), 6.91 (dd, *J* = 8.0, 1.4 Hz, 6H), 6.86 (dd, *J* = 5.1, 3.0 Hz, 1 H), 6.77 (t, *J* = 7.6 Hz, 6H), 6.74 (m, 3H), 4.53 (br s, 1H), 3.20 (br s, 1H), 2.20 (s, 3H), 1.20 (br s, 3H), 0.93 (s, 3H), 0.56 (br s, 6H), -0.67 ppm (br s, 3H); ¹³C NMR (100 MHz, CD₂Cl₂): δ = 300.2, 157.5, 151.4, 150.2, 149.3, 144.6, 143.2, 141.4, 140.4, 138.3, 137.4, 136.7, 136.3, 135.4, 135.3, 130.5, 129.9, 129.8, 128.6, 128.3, 128.0, 127.8, 127.5, 127.4, 127.2, 127.0, 126.9, 126.1, 125.4, 124.5, 124.1, 54.6, 31.2, 28.3, 28.2, 22.6 ppm. IR (Film, cm⁻¹): 3065, 2963, 2866, 1517, 1426, 1333, 1285, 1110, 971, 918, 871, 742, 699.

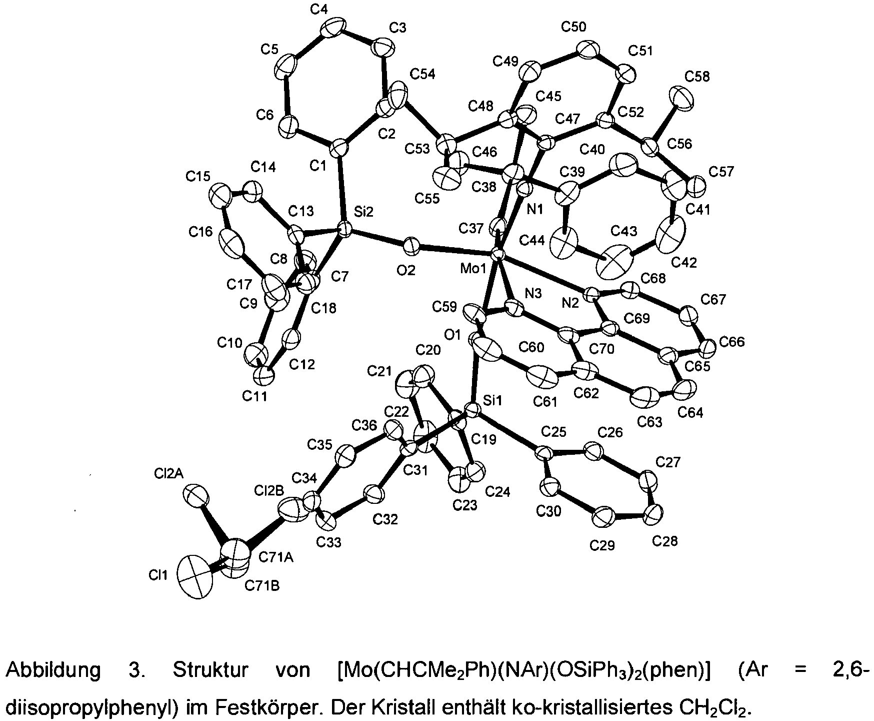

### [Mo(CHCMe₂Ph)(NAr)((R)-(+)-tBu₂Me₄(biphen))(bipy)] (Ar = 2,6-diisopropylphenyl).

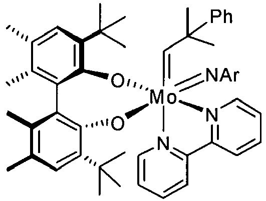

Eine Lösung von [Mo(CHCMe₂Ph)(NAr)((*R*)-(+)-*tert*-Bu₂Me₄(biphen)] (316 mg, 418 µmol) in Et₂O (3 mL) wurde zu einer Lösung von 2,2'-Bipyridin (65 mg, 418 µmol) in Et₂O (2 mL) zugegeben und das Gemisch 1 h bei Raumtemperatur gerührt. Anschließend wurde das Lösungsmittel abgezogen und der Rückstand durch Umkristallisation aus Pentan bei - 40°C gereinigt. Nach Trocknen im Vakuum erhält man den gewünschten Komplex als orange-roten Feststoff (282 mg, 74%). ¹H NMR (400 MHz, CD₂Cl₂): δ = 13.03 (s, 1 H), 8.62 (dd, *J* = 5.2, 1.8 Hz, 1 H), 7.87 (d, *J* = 8.0 Hz, 1 H), 7.79 (d, *J* = 8.2 Hz, 1 H), 7.74-7.68 (m, 2H), 7.56 (dd, *J* = 8.5, 1.2 Hz, 2H), 7.36-7.31 (m, 3H), 7.19 (tt, *J* = 7.2, 1.2 Hz, 1H), 6.98 (s, 1H), 6.94 (ddd, *J* = 7.4, 5.2, 1.1 Hz, 1H), 6.83 (ddd, *J* = 7.4, 5.4, 1.2 Hz, 1 H), 6.79-6.74 (m, 3H), 6.35 (s, 1H), 4.18 (br s, 1H), 3.65 (br s, 1H), 2.24 (s, 3H), 2.12 (s, 3H), 1.73 (s, 3H), 1.57 (s, 3H), 1.54 (s, 3H), 1.49 (s, 9H), 1.28 (br s, 3H), 1.04 (s, 6H), 0.71 (br s, 3H), 0.68 (s, 9H), -0.01 ppm (br s, 3H); ¹³C NMR (100 MHz, CD₂Cl₂): δ = 305.3, 168.6, 162.2, 157.9, 154.4, 151.9, 151.6, 150.7, 150.4, 149.5, 148.8, 148.6, 146.9, 138.7, 138.4, 137.2, 133.8, 133.6, 133.2, 132.4, 132.0, 131.5, 129.2, 128.4, 127.2, 126.4, 125.7, 125.4, 125.3, 125.0, 124.9, 124.8, 122.6, 122.0, 121.2, 35.0, 34.8, 34.5, 32.1, 32.0, 31.5, 29.7, 27.4, 26.4, 25.2, 24.2, 22.6, 20.3, 20.1, 19.9, 16.4, 16.0, 15.8, 14.2 ppm; IR (Film, cm⁻¹): 2920, 2862, 1738, 1595, 1408, 1261, 1040, 974, 859, 798, 756, 702.

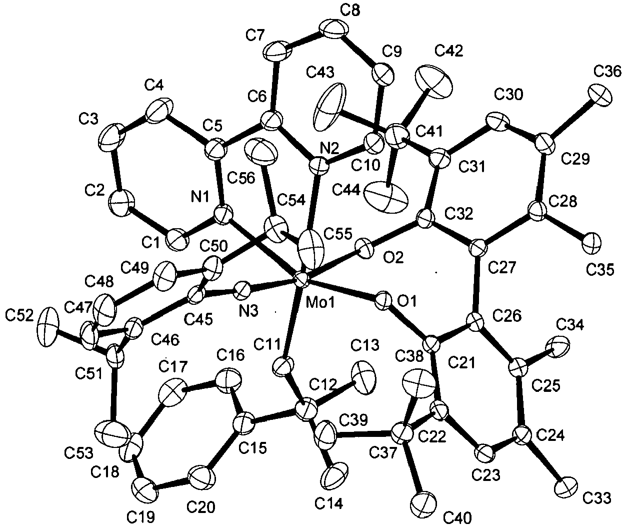

Abbildung 4. Struktur von [Mo(CHCMe₂Ph)(NAr)((*R*)-(+)-*t*Bu₂Me₄(biphen))(bipy)] (Ar = 2,6-diisopropylphenyl) im Festkörper.

**Ringschluss-Olefin Metathese mit Hilfe von [Mo(CHCMe₂Ph)(NAr)(OCMe(CF₃)₂)₂(phen)] in Gegenwart von ZnCl₂ (Ar = 2,6-diisopropylphenyl)).** Eine Lösung von [Mo(CHCMe₂Ph)(NAr)(OCMe(CF₃)₂)₂(phen)] (24 mg, 25 µmol, 5 mol%) and wasserfreiem ZnCl₂ (3.4 mg, 25 µmol) in Toluol (2.5 mL) wurde unter Argon für 30 min auf 100°C erhitzt. Nach dieser Aktivierungsphase wurde das Gemisch auf Raumtempertur abgekühlt bevor Diethyl 2,2-diallylmalonat (121 µL, 500 µmol) zugegeben wurde. Danach wurde weitere 30 min bei Raumtemperatur gerührt, anschließend das Lösungsmittel abgezogen und der Rückstand säulenchromatographisch gereinigt. Man erhielt Diethylcyclopent-3-en-1,1-dicarboxylat als farbloses Öl (104 mg, 98%). ¹H NMR (400 MHz, CDCl₃): δ = 5.59 (s, 2H), 4.19 (q, *J* = 7.1 Hz, 4H), 3.00 (s, 4H), 1.24 ppm (t, *J* = 7.1 Hz, 6H); ¹³C NMR (100 MHz, CDCl₃): δ = 172.4, 127.9, 61.6, 59.0, 41.0, 14.2 ppm; IR (Film, cm⁻¹): 3463, 2982, 2872, 1728, 1446, 1366, 1248, 1179, 1060, 1015, 951, 695; MS (EI) *m*/*z* (%): 212 [M⁺] (25), 166 (45), 138 (100), 111 (41), 93 (39), 79 (56), 66 (76), 55 (5), 39 (16), 29 (97); HRMS (ESI): *m*/*z:* berechnet für C₁₁H₁₆O₄ + Na: 235.0946; gefunden: 235.0941. Die erhaltenen Daten entsprechen jenen der Literatur (T. Kirkland, R. H. Grubbs, *J. Org. Chem.* **1997,** *62*, 7310-7318).

### Diethyl-3,4-dimethylcyclopent-3-en-1,1-dicarboxylat.

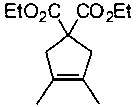

Analog hergestellt als farbloses Öl.¹H NMR (400 MHz, CDCl₃): δ = 4.18 (q, *J* = 7.1 Hz, 4H), 2.92 (s, 4H), 1.58 (s, 6H), 1.23 ppm (t, *J* = 7.1 Hz, 6H); ¹³C NMR (100 MHz, CDCl₃): δ = 172.7, 128.2, 122.9, 61.5, 57.3, 46.0, 28.1, 22.6, 14.2, 13.4 ppm; IR (Film, cm⁻¹): 3462, 2970, 2859, 1729, 1443, 1366, 1246, 1180, 1071, 1019; MS (EI) *m*/*z* (%): 240 [M⁺] (26), 195 (7), 166 (100), 138 (11), 121 (23), 107 (15), 93 (42), 79 (17), 29 (30); HRMS (ESI): *m*/*z*: berechnet für C₁₃H₂₀O₄ + Na: 263.1259; gefunden: 263.1254. Die erhaltenen Daten entsprechen jenen der Literatur (T. Kirkland, R. H. Grubbs, J. Org. Chem. 1997, 62, 7310-7318).

### Diethyl-3-tert-butylcyclopent-3-en-1,1-dicarboxylat.

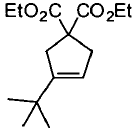

Analog hergestellt als farbloses Öl. ¹H NMR (400 MHz, CDCl₃): δ = 5.17 (s, 1 H), 4.18 (q, *J* = 7.1 Hz, 4H), 2.94 (s, 4H), 1.23 (t, *J* = 7.1 Hz, 6H), 1.04 ppm (s, 9H);¹³C NMR (100 MHz, CDCl₃): δ = 172.4, 151.1, 117.6, 61.5, 59.6, 40.3, 39.9, 32.8, 29.0, 14.2 ppm; IR (Film, cm⁻¹): 3462, 2964, 2869, 1730, 1446, 1364, 1245, 1183, 1060, 1024, 815, 737; MS (EI) *m*/*z* (%): 268 [M⁺] (24), 223 (8), 194 (58), 179 (82), 151 (14), 138 (20), 133 (22), 121 (100), 107 (82), 91 (21), 79 (12), 65 (7), 57 (26), 41 (14), 29 (58); HRMS (ESI): *m*/*z:* berechnet für C₁₅H₂₄O₄ + Na: 291.1572; gefunden: 291.1567. Die erhaltenen Daten entsprechen jenen der Literatur (T. Kirkland, R. H. Grubbs, *J. Org. Chem.* **1997**, *62*, 7310-7318).

### Diethyl-3-methylcyclohept-3-en-1,1-dicarboxylat.

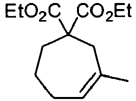

Analog hergestellt als farbloses Öl. ¹H NMR (400 MHz, CDCl₃): δ = 5.57 (t, *J* = 6.2 Hz, 1H), 4.16 (q, *J* = 7.1 Hz, 4H), 2.64 (s, 2H), 2.19-2.16 (m, 2H), 2.07-2.03 (m, 2H), 1.75 (s, 3H), 1.65-1.59 (m, 2H), 1.23 ppm (t, *J* = 7.1 Hz, 6H); ¹³C NMR (100 MHz, CDCl₃): δ = 172.0, 135.2, 127.7, 61.2, 55.8, 37.6, 36.9, 28.0, 26.7, 22.9, 14.2 ppm; IR (Film, cm⁻¹): 3463, 2970, 2862, 1729, 1446, 1366, 1310, 1217, 1090, 1056, 1030; MS (EI) *m*/*z* (%): 254 [M⁺] (15), 208 (20), 180 (22), 173 (100), 163 (14), 152 (7), 135 (15), 127 (28), 107 (33), 91 (9), 79 (11); HRMS (ESI): *m*/*z*: berechnet für C₁₄H₂₂O₄ + Na: 277.1416; gefunden: 277.1410. Die erhaltenen Daten entsprechen jenen der Literatur (T. Kirkland, R. H. Grubbs, J. Org. Chem. 1997, 62, 7310-7318).

### 2,2-Dimethyl-6-phenyl-1-oxa-2-silacyclohex-3-en.

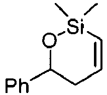

Analog hergestellt als farbloses Öl; die Reinigung erfolgte durch Kugelrohrdestillation (bp = 93-95°C, 0.4 Torr). ¹H NMR (400 MHz, CDCl₃): δ = 7.43(dd, *J* = 8.8, 1.6 Hz, 2H), 7.37 (td, *J* = 8.8, 1.6 Hz, 2H), 7.27 (tt, *J* = 8.8, 1.6 Hz, 1 H), 6.86 (ddd, *J* = 14.0, 6.0, 2.4 Hz, 1 H), 5.89 (ddd, *J* = 14.0, 2.8, 0.8 Hz, 1 H), 5.02 (dd, *J* = 10.0, 3.6 Hz, 1 H), 2.47-2.33 (m, 2H), 0.28 (s, 3H), 0.27 ppm (s, 3H); ¹³C NMR (100 MHz, CDCl₃): δ = 147.2, 144.6, 128.5, 127.6, 127.3, 125.7, 73.5, 39.1, 0.0, -0.4 ppm; IR (Film, cm⁻¹): 2958, 2855, 1587, 1249, 1062, 955, 833, 785, 695; MS (EI) *m*/*z* (%): 204 [M⁺] (43), 189 (5), 130 (100), 111 (5), 98 (40), 83 (25), 75 (6), 61 (5); HRMS (ESI): *m*/*z:* berechnet für C₁₂H₁₆OSi: 204.0970; gefunden: 204.0970. Die erhaltenen Daten entsprechen jenen der Literatur (S. E. Denmark, S.-M. Yang, Org. Lett. 2001, 3, 1749-1752).

### 2-Benzylbenzofuran.

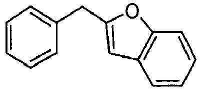

Analog hergestellt als farbloses Öl. ¹H NMR (400 MHz, CDCl₃): δ = 7.43-7.41 (m, 1H), 7.37-7.35 (m, 1H), 7.30-7.24 (m, 4H), 7.23-7.10 (m, 3H), 6.32 (s, 1H), 4.06 ppm (s, 2H);¹³C NMR (100 MHz, CDCl₃): δ = 157.9, 155.1, 137.4, 129.1, 128.8, 126.9, 123.6, 122.7, 120.5, 111.1, 103.5, 35.1 ppm; IR (Film, cm⁻¹): 3029, 2906, 1738, 1585, 1495, 1453, 1251, 1163, 952, 791, 739, 701; MS (EI) *m*/*z* (%): 208 [M⁺] (100), 189 (4), 178 (14), 165 (3), 152 (3), 131 (32), 115 (3), 104 (4), 89 (7), 77 (5), 51 (3); HRMS (ESI): *m*/*z*: berechnet für C₁₅H₁₂O: 208.0888; gefunden: 208.0888. Die erhaltenen Daten entsprechen jenen der Literatur (O. Fujimura, G. C. Fu, R. H. Grubbs J. Org. Chem. 1994, 59, 4029-4031).

### (3R, 4R)-3-Azido-4-benzyloxy-2,3,4,7-tetrahydroazepin-1-carbonsäure-tertbutylester.

Eine Lösung von Mo(CHCMe₂Ph)(NAr)(OCMe(CF₃)₂)₂(phen) (8 mg, 9 µmol, 5 mol%) und

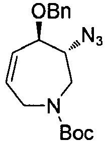

ZnCl₂ (1.2 mg, 9 µmol) in CH₂Cl₂ (18 mL) wird für 30 min auf 100°C erhitzt. Anschließend wird (2R,3R)-Allyl-(2-azido-3-benzyloxy-pent-4-enyl)-carbaminsäure-*tert*-butylester (66 mg, 0.177 mmol) zugegeben und das Gemisch für 30 min zum Rückfluss erhitzt. Nach Entfernen des Lösungsmittels wird der Rückstand säulenchromatographisch an Kieselgel (Hexan/Ethylacetat, 20:1) gereinigt. ¹H NMR (300 MHz, CD₂Cl₂): δ = 7.40-7.29 (m, 5H), 5.76 (br s, 2H), 4.67 (d, *J =* 11.5 Hz, 1H), 4.55 (d, *J* = 11.5 Hz, 1H), 427-3.97 (m, 2H), 3.85-3.54 (m, 4H), 1.45 (s, 9H); ¹³C NMR (75 MHz, CD₂Cl₂): δ = 155.1, 138.3, 131.1, 129.8, 128.7, 128.3, 128.1, 80.5, 79.1, 72.3, 64.0, 48.8, 47.5, 28.4; [α]²⁰_{D}= -26.3 (2.65, CH₂Cl₂); IR (Film, cm⁻¹): 3065, 3031, 2976, 2927, 2857, 2108, 1699, 1657, 1496, 1456, 1393, 1366, 1248, 1168, 1135, 1095, 737, 698 cm⁻¹; MS (EI) *m*/*z*: 344 ([M⁺], < 1), 260 (1), 225 (2),186 (1),180 (3), 169 (14), 160 (6), 125 (11), 91 (100), 57 (57); HRMS (ESI) (C₁₈H₂₄N₄O₃⁺ H) berechnet: 345.1927, gefunden: 345.1921; C₁₈H₂₄N₄O₃ (344.41) berechnet: C 62.77, H 7.02, N 16.27, gefunden: C 62.72, H 6.94, N 16.19. Die erhaltenen Daten entsprechen jenen der Literatur (A. Fürstner, O. R Thiel, J. Org. Chem. 2000, 65, 1738)

**Kinetische Resolution unter Verwendung von [Mo(CHCMe₂Ph)(NAr)((*R*)-(+)-*t*Bu₂Me₄(biphen))(bipy)] in Gegenwart von ZnCl₂ (Ar = 2,6-diisopropylphenyl).** Eine Lösung von [Mo(CHCMe₂Ph)(NAr)((R)-(+)-*t*Bu₂Me₄(biphen))(bipy)] (31 mg, 33 µmol, 5 mol%) und ZnCl₂ (4.5 mg, 33 µmol) in Toluol (2.6 mL) wurde 30 min bei 80°C gerührt. Nach dieser Aktivierungsphase wurde das Gemisch auf Raumtemperatur abgekühlt und eine Lösung von (6*E*)-6-Methyl-5-*tert*-butyldimethylsiloxy-1,6-oktadien (168 mg, 660 µmol) in Toluol (4 mL) zugegeben. Das Gemisch wurde im geschlossenen Gefäß 80 min gerührt bevor die Umsetzung durch Zugabe von MeOH (3 mL) abgebrochen wurde. Das Gemisch wurde eingeengt und der braune Rückstand chromatographisch gereinigt. Die erhaltenen Produkte haben folgende spektroskopische Eigenschaften:

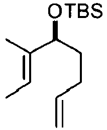

### (6E)-6-Methyl-5-tert-butyldimethylsiloxy-1,6-oktadien.

ee = 90% (bestimmt durch GC an chiraler Phase: Ivadex-1/PS086 (Dimethyl-pentyl-ß-cyclodextrin, 25 m)); ¹H NMR (400 MHz, CDCl₃): δ = 5.87-5.77 (m, 1 H), 5.38-5.32 (m, 1 H), 5.02-4.91 (m, 2H), 3.95 (t, *J* = 7.1 Hz, 1H), 2.10-1.91 (m, 2H), 1.66-1.44 (m, 8H), 0.87 (s, 9H), 0.02 (s, 3H), -0.03 (s, 3H); ¹³C NMR (100 MHz, CDCl₃): δ = 139.1, 138.4, 119.8, 114.3, 78.2, 35.7, 30.3, 26.0, 18.4, 13.1, 10.9, -4.5, -4.8; IR (Film, cm⁻¹): 2929, 2858, 1738, 1641, 1472, 1361, 1252, 1217, 1071, 909, 833, 772, 665; MS (EI) *m*/*z* (%): 254 [M⁺] (1); 239 (2), 197 (64), 155 (8), 141 (3), 127 (4), 75 (100), 67 (3), 59 (4), 41 (7); HRMS (ESI): *m*/*z:* berechnet für C₁₅H₃₀OSi + H: 255.2144; gefunden: 255.2144.

### 2-Methyl-1-tert-butyldimethylsiloxy-2-cyclopenten.

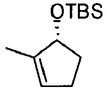

ee = 60% (bestimmt durch GC an chiraler Phase: BGB-176/SE-52 (2,3-Dimethyl-6-*tert-*butyldimethylsilyl-β-cyclodextrin, 30 m)); ¹H NMR (400 MHz, CDCl₃): δ = 5.47-5.45 (m, 1 H), 4.66-4.63 (m, 1 H), 2.40-2.32 (m, 1 H), 2.28-2.20 (m, 1 H), 2.18-2.10 (m, 1 H), 1.71 (s, 3H), 1.70-1.63 (m, 1H), 0.92 (s, 9H), 0.10 (s, 3H), 0.09 (s, 3H); ¹³C NMR (100 MHz, CDCl₃): δ = 142.2, 126.9, 80.3, 34.5, 29.8, 26.1, 14.0, -4.3, -4.6; IR (Film, cm⁻¹): 2930, 2856, 1462, 1354, 1249, 1080, 992, 884, 833, 772, 669; MS (EI) *m*/*z* (%): 212 [M⁺] (9), 197 (2), 137 (3), 155 (32), 75 (100), 59 (3); HRMS (ESI): *m*/*z:* berechnet für C₁₂H₂₄OSi: 212.1596; gefunden: 212.1596.

## Patentansprüche

1. Metallorganische Verbindungen der allgemeinen Formel I 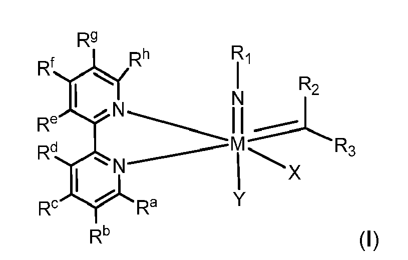 in der
M für Mo oder W steht,
R¹ für C₁-C₁₂-Alkyl, 5- bis 18-gliedriges Aryl, die ihrerseits mit einem oder mehreren gleichen oder verschiedenen C₁-C₁₂-Alkyl, 5- bis 18-gliedriges Aryl, C₁-C₁₂-Alkyloxy, Di(C₁-C₄-Alkyl)amino, Halogen, Trifluormethyl, Cyano, Nitro-Resten substituiert sein können, steht,
die Substituenten X, Y gleich oder verschieden sein können und unabhängig voneinander ausgewählt werden können aus: Halogen, Methansulfonyloxy, Trifluormethansulfonyloxy, Benzolsulfonyloxy, Toluolsulfonyloxy, Pyrrolyl, C₁-C₁₂-Alkyloxy, 5- bis 18-gliedriges Aryloxy, Tri(C₁-C₁₂-Alkyl)silyloxy, Di(C₁-C₁₂-Alkyl)(C₆-C₁₈-Aryl)silyloxy, (C₁-C₁₂-Alkyl)di(C₆-C₁₈-Aryl)silyloxy, Tris(C₁-C₁₂-Alkyloxy)silyloxy, die ihrerseits mit einem oder mehreren gleichen oder verschiedenen C₁-C₁₂-Alkyl, 5- bis 18-gliedriges Aryl, C₁-C₁₂-Alkoxy, Di-(C₁-C₄-Alkyl)amino, Halogen, Trifluormethyl, Cyano, Nitro Resten substituiert sein können, ,
R² für H, C₁-C₁₂-Alkyl, 5- bis 18-gliedriges Aryl, die ihrerseits mit einem oder mehreren gleichen oder verschiedenen C₁-C₁₂-Alkyl, 5- bis 18-gliedriges Aryl, C₁-C₁₂-Alkyloxy, Di(C₁-C₄-Alkyl)amino, Halogen, Trifluormethyl, Cyano, Nitro Resten substituiert sein können, steht,
R³ für C₁-C₁₂-Alkyl, 5- bis 18-gliedriges Aryl, die ihrerseits mit einem oder mehreren gleichen oder verschiedenen C₁-C₁₂-Alkyl, 5- bis 18-gliedriges Aryl, C₁-C₁₂-Alkyloxy, Di(C₁-C₄-Alkyl)amino, Halogen, Trifluormethyl, Cyano, Nitro Resten substituiert sein können, steht,
R^{a}, R^{b}, R^{c}, R^{d}, R^{e}, R^{f}, R^{g}, R^{h} gleich oder verschieden sein können und unabhängig voneinander ausgewählt werden können aus H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können, oder die Reste R^{d} und R^{e} unter Bildung eines 5 bis 8 gliedrigen Ringes mit einander verbunden sind.

2. Metallkomplexe nach Anspruch 1, **dadurch gekennzeichnet, dass** R^{d} und R^{e} jeweils für einen Rest R⁴⁸ stehen, der CR⁴⁹R⁵⁰, CR⁵¹=CR⁵², CR⁵³R⁵⁴-CR⁵⁵R⁵⁶, CR⁵⁷R⁵⁸-CR⁵⁹R⁶⁰-CR⁶¹R⁶², CR⁶²R⁶³-CR⁶⁴R⁶⁵R⁶⁷, CR⁶⁹R⁷⁰-CR⁷¹R⁷²-CR⁷³R⁷⁴-CR⁷⁵CR⁷⁶, CR⁷⁷=CR⁷⁸-CR⁷⁹R⁸⁰-CR⁸¹CR⁸² und/oder CR⁸³R⁸⁴-CR⁸⁵=CR⁸⁷-CR⁸⁸CR⁸⁹ bedeutet, in welchem R⁴⁹, R⁵⁰, R⁵¹, R⁵², R⁵³, R⁵⁴, R⁵⁵, R⁵⁶, R⁵⁷, R⁵⁸, R⁵⁹, R⁶⁰, R⁶¹, R⁶², R⁶³, R⁶⁴, R⁶⁵, R⁶⁶, R⁶⁷, R⁶⁸, R⁶⁹, R⁷⁰, R⁷¹, R⁷², R⁷³, R⁷⁴, R⁷⁵, R⁷⁶, R⁷⁷, R⁷⁸, R⁷⁹, R⁸⁰, R⁸¹, R⁸², R⁸³, R⁸⁴, R⁸⁵, R⁸⁶, R⁸⁷, R⁸⁸ und R⁸⁹ jeweils unabhängig voneinander ausgewählt sind und die gleiche Bedeutung haben können wie R^{a}.

3. Metallorganische Verbindungen nach Anspruch 1 oder Anspruch 2, **dadurch gekennzeichnet, dass** sie Verbindungen der allgemeinen Formel 2 darstellen: 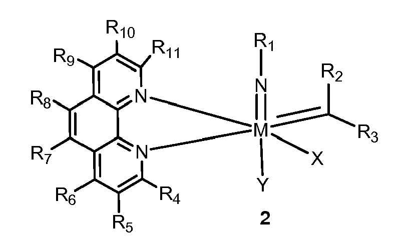 in der
R¹, R², R³, X und Y wie in Anspruch 1 definiert sind und
R⁴, R⁵, R⁶, R⁷, R⁸, R⁹, R¹⁰, R¹¹ gleich oder verschieden sein können und unabhängig voneinander ausgewählt werden können aus H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

4. Metallorganische Verbindungen nach einem der Ansprüche 1 bis 3, **dadurch gekennzeichnet, dass** sie Verbindungen der allgemeinen Formel 9 darstellen: 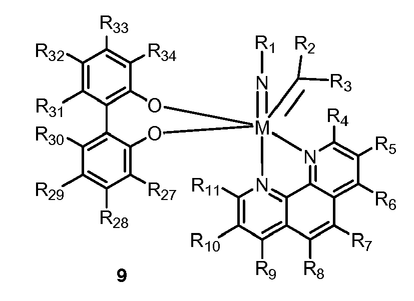 worin
M, R¹, R², R³ und R⁴, R⁵, R⁶, R⁷, R⁸, R⁹, R¹⁰, R¹¹ wie oben definiert sind,
R²⁷, R²⁸, R²⁹, R³⁰, R³¹, R³², R³³, R³⁴ gleich oder verschieden sein können und unabhängig voneinander ausgewählt werden können aus: H, Halogen, Nitro, Cyano, Trifluormethyl, C₁-C₁₂ Alkoxycarbonyl, C₁-C₁₂ Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

5. Metallorganische Verbindungen nach einem der Ansprüche 1 bis 4, **dadurch gekennzeichnet, dass** sie Verbindungen der allgemeinen Formel 10 darstellen: 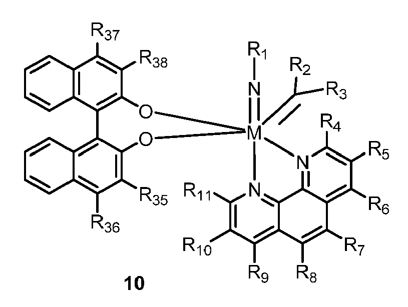 worin
M, R¹, R², R³, R⁴, R⁵, R⁶, R⁷, R⁸, R⁹, R¹⁰ und R¹¹ wie in den Ansprüchen 1 und 3 definiert sind und
R³⁵, R³⁶, R³⁷, R³⁸ gleich oder verschieden sein können und unabhängig voneinander ausgewählt werden können aus: H, Halogen, Nitro, Cyano, Trifluormethyl, C₁-C₁₂-Alkyl, 5- bis 18-gliedriges Aryl, C₁-C₁₂-Alkyloxy, Di-(C₁-C₄-Alkyl)amino, Tri(C₁-C₁₂-Alkyl)silyl, Di(C₁-C₁₂-Alkyl)(C₆-C₁₈-Aryl)silyl, (C₁-C₁₂-Alkyl)di(C₆-C₁₈-Aryl)silyl, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

6. Metallorganische Verbindungen nach Anspruch 1 oder Anspruch 2, **dadurch gekennzeichnet, dass** sie Verbindungen der allgemeinen Formel 17 darstellen: 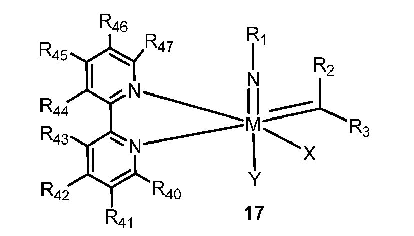
in der R¹, R², R³, X und Y wie in Anspruch 1 definiert sind und
R⁴⁰, R⁴¹, R⁴², R⁴³, R⁴⁴, R⁴⁵, R⁴⁶, R⁴⁷ gleich oder verschieden sein können und unabhängig voneinander ausgewählt werden können aus H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

7. Metallorganische Verbindungen nach einem der Ansprüche 1, 2 oder 4, **dadurch gekennzeichnet, dass** sie Verbindungen der allgemeinen Formel 24 darstellen: 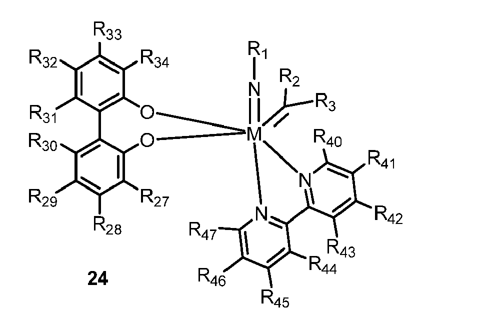 worin
M, R¹, R² und R³ sowie R⁴⁰, R⁴¹, R⁴², R⁴³, R⁴⁴, R⁴⁵, R⁴⁶ und R⁴⁷ wie in den Ansprüchen 1 und 4 definiert sind und
R²⁷, R²⁸, R²⁹, R³⁰, R³¹, R³², R³³, R³⁴ gleich oder verschieden sein können und unabhängig voneinander ausgewählt werden können aus: H, Halogen, Nitro, Cyano, Trifluormethyl, C₁-C₁₂-Alkoxycarbonyl, C₁-C₁₂-Alkyl, 5- bis 18-gliedriges Aryl, C₁-C₁₂-Alkyloxy, Di-(C₁-C₄-Alkyl)amino, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

8. Metallorganische Verbindungen nach einem der Ansprüche 1, 2, 4 oder 7, **dadurch gekennzeichnet, dass** sie Verbindungen der allgemeinen Formel 25 darstellen: 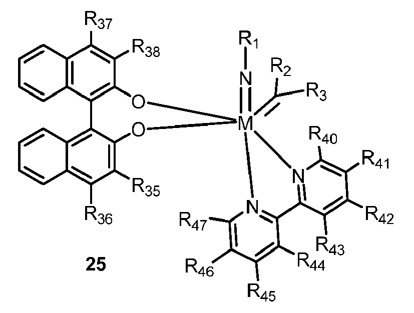 worin
M, R¹, R² und R³ sowie R⁴⁰, R⁴¹, R⁴², R⁴³, R⁴⁴, R⁴⁵, R⁴⁶ und R⁴⁷ wie in den Ansprüchen 1 und 4 definiert sind und
R³⁵, R³⁶, R³⁷, R³⁸ gleich oder verschieden sein können und unabhängig voneinander ausgewählt werden können aus: H, Halogen, Nitro, Cyano, Trifluormethyl, C₁-C₁₂-Alkyl, 5- bis 18-gliedriges Aryl, C₁-C₁₂-Alkyloxy, Di-(C₁-C₄-Alkyl)amino, Tri(C₁-C₁₂-Alkyl)silyl, Di(C₁-C₁₂-Alkyl)(C₆-C₁₈-Aryl)silyl, (C₁-C₁₂-Alkyl)di(C₆-C₁₈-Aryl)silyl, die ihrerseits mit einem oder mehreren gleichen oder verschiedenen C₁-C₁₂-Alkyl, 5- bis 18-gliedriges Aryl, C₁-C₁₂-Alkyloxy, Di-(C₁-C₄-Alkyl)amino, Halogen, Trifluormethyl, Cyano, Nitro Resten substituiert sein können.

9. Verfahren zur Herstellung der Metallkomplexe mit der allgemeinen Formel I 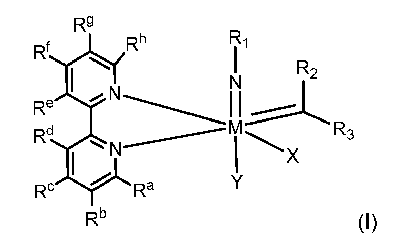 worin M, X, Y, R¹, R², R³, R^{a}, R^{b}, R^{c}, R^{d}, R^{e}, R^{f}, R^{g}, R^{h} wie in Anspruch 1 definiert sind,
bei dem eine Verbindung der Formel 32 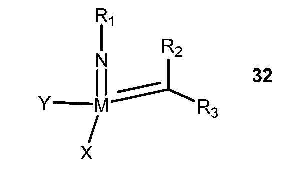 worin M, X, Y, R₁, R₂, R₃ wie in Anspruch 1 definiert sind,
mit einer Verbindung der allgemeinen Formel II 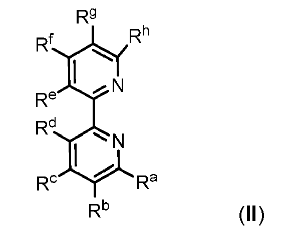 worin R^{a}, R^{b}, R^{c}, R^{d}, R^{e}, R^{f}, R^{g}, R^{h} wie in Anspruch 1 definiert sind,
umgesetzt wird.

10. Verwendung der Verbindungen mit der allgemeinen Formel I 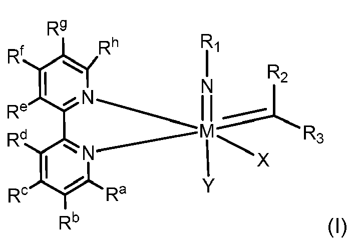 worin M, X, Y, R¹, R², R³, R^{a}, R^{b}, R^{c}, R^{d}, R^{e}, R^{f}, R^{g}, R^{h} wie in Anspruch 1 definiert
sind, als Präkatalysatoren für Olefinmetathese-Reaktionen.

## Claims

1. Metal organic compounds of the general formula I 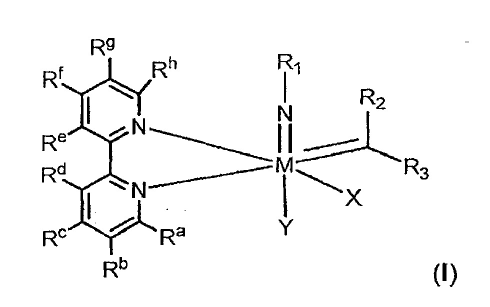 wherein
M is Mo or W,
R¹ is C₁-C₁₂-alky, 5- to 18-membered aryl, which in turn may be substituted with one or more of C₁-C₁₂-alkyl, 5- to 18-membered aryl, C₁-C₁₂-alkyloxy, di(C₁-C₄-alkyl)amino, halogen, trifluoromethyl, cyano, nitro residues, which may be the same or may be different,
the substituents X, Y may be the same or may be different and may be inde-pendently selected from one another from: halogen, methanesulfonyloxy, trifluoro-methanesulfonyloxy, benzenesulfonyloxy, toluenesulfonyloxy, pyrrolyl, C₁-C₁₂-alkyloxy, 5- to 18-membered aryloxy, tri(C₁-C₁₂-alkyl)silyloxy, di(C₁-C₁₂-alkyl)(C₆-C₁₈-aryl)silyloxy, (C₁-C₁₂-alkyl)di(C₆-C₁₈-alryl)silyloxy, tris(C₁-C₁₂-alkyloxy)silyloxy, which in turn may be substituted with one or more of C₁-C₁₂-alkyl, 5- to 18-membered aryl, C₁-C₁₂-alkoxy, di-(C₁-C₄-alkyl)amino, halogen, trifluoromethyl, cyano, nitro residues, which may be the same or may be different,
R² is H, C₁-C₁₂-alkyl, 5- to 18-membered aryl, which in turn may be substituted with one or more of C₁-C₁₂-alkyl, 5- to 18-membered aryl, C₁-C₁₂-alkyloxy, di(C₁-C₄-alkyl)amino, halogen, trifluoromethyl, cyano, nitro residues, which may be the same or may be different,
R³ is C₁-C₁₂-alkyl, 5- to 18-membered aryl, which in turn may be substituted with one or more of C₁-C₁₂-alkyl, 5- to 18-membered aryl, C₁-C₁₂-alkyloxy, di(C₁-C₄-alkyl)amino, halogen, trifluoromethyl, cyano, nitro residues, which may be the same or may be different,
R^{a}, R^{b}, R^{c}, R^{d}, R^{e}, R^{f}, R^{g}, R^{h} may be the same or may be different and may be inde-pendently selected from one another from H, halogen, nitro, cyano, trifluoromethyl, C₁-C₁₂-alkoxycarbonyl, C₁-C₁₂-alkyl, 5- to 18-membered aryl, C₁-C₁₂-alkyloxy, di-(C₁-C₄-alkyl)amino, which in turn may be substituted with one or more of C₁-C₁₂-alkyl, 5- to 18-membered aryl, C₁-C₁₂-alkyloxy, di-(C₁-C₄-alkyl)amino, halogen, tri-fluoromethyl, cyano, nitro residues, which may be the same or may be different, or residues R^{d} and R^{e} are connected to one other while forming a 5- to 8-membered ring.

2. Metal complexes according to claim 1, **characterized in that** R^{d} and R^{e} are a residue R⁴⁸, respectively, which is CR⁴⁹R⁵⁰, CR⁵¹=CR⁵², CR⁵³R⁵⁴-CR⁵⁵R⁵⁶, CR⁵⁷R⁵⁸-CR⁵⁹R⁶⁰-CR⁶¹R⁶², CR⁶²R⁶³=CR⁶⁴R⁶⁵R⁶⁷, CR⁶⁹R⁷⁰-CR⁷¹R⁷²-CR⁷³R⁷⁴-CR⁷⁵CR⁷⁶, CR⁷⁷=CR⁷⁸-CR⁷⁹R⁸⁰-CR⁸¹CR⁸² and/or CR⁸³R⁸⁴-CR⁸⁵=CR⁸⁷-CR⁸⁸CR⁸⁹ in which R⁴⁹, R⁵⁰ R⁵¹, R⁵², R⁵³, R⁵⁴, R⁵⁵, R⁵⁶, R⁵⁷, R⁵⁸, R⁵⁹, R⁶⁰, R⁶¹, R⁶², R⁶³, R⁶⁴, R⁶⁵, R⁶⁶, R⁶⁷, R⁶⁸, R⁶⁹, R⁷⁰, R⁷¹, R⁷², R⁷³, R⁷⁴, R⁷⁵, R⁷⁶, R⁷⁷, R⁷⁸, R⁷⁹, R⁸⁰, R⁸¹, R⁸², R⁸³, R⁸⁴, R⁸⁵, R⁸⁶, R⁸⁷, R⁸⁸ and R⁸⁹ are independently selected from one another, respectively, and may have the same meaning as R^{a}.

3. Metal organic complexes according to claim 1 or claim 2, **characterized in that** they represent compounds of the general formula 2: 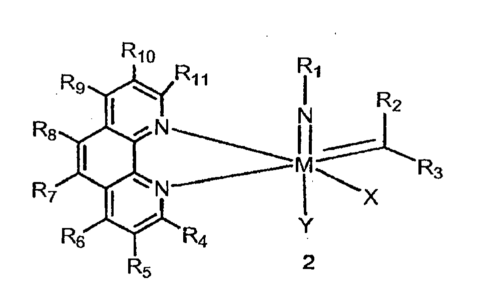 in which
R¹, R², R³, X and Y are defined in claim 1, and
R⁴, R⁵, R⁶, R⁷, R⁸, R⁹, R¹⁰, R¹¹ may be the same or may be different and may be independently selected from one another from H, halogen, nitro, cyano, trifluorome-thyl, C₁-C₁₂-alkoxycabonyl, C₁-C₁₂-alkyl, 5- to 18-membered aryl, C₁-C₁₂-alkyloxy, di-(C₁-C₄-alkyl)amino, which in turn may be substituted with one or more of C₁-C₁₂-alkyl, 5- to 18-membered aryl, C₁-C₁₂-alkyloxy, di-(C₁-C₄-alkyl)amino, halogen, tri-fluoromethyl, cyano, nitro residues, which may be the same or may be different.

4. Metal organic compounds according to one of claims 1 to 3, **characterized in that** they represent compounds of the general formula 9: 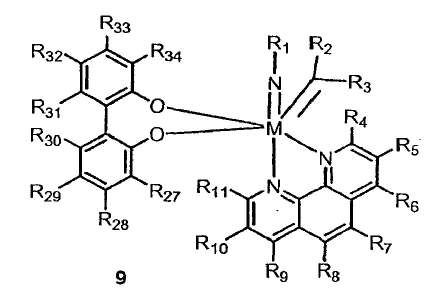 wherein
M, R¹, R², R³ and R⁴, R⁵, R⁶, R⁷, R⁸, R⁹, R¹⁰, R¹¹ are defined above,
R²⁷, R²⁸, R²⁹, R³⁰, R³¹, R³², R³³, R³⁴ may be the same or may be different and may be independently selected from one another from: H, halogen, nitro, cyano, trifluo-romethyl, C₁-C₁₂-alkoxycabonyl, C₁-C₁₂-alkyl, 5- to 18-membered aryl, C₁-C₁₂-alkyloxy, di-(C₁-C₄-alkyl)amino, which in turn may be substituted with one or more of C₁-C₁₂-alkyl, 5- to 18-membered aryl, C₁-C₁₂-alkyloxy, di-(C₁-C₄-alkyl)amino, hal-ogen, trifluoromethyl, cyano, nitro residues, which may be the same or may be different.

5. Metal organic compounds according to one of claims 1 to 4, **characterized in that** they represent compounds of the general formula 10: 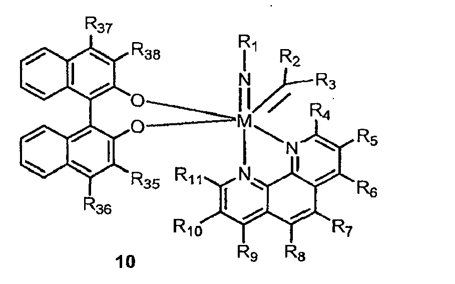 wherein
M, R¹, R², R³, R⁴, R⁵, R⁶, R⁷, R⁸, R⁹, R¹⁰ and R¹¹ are defined in claims 1 and 3 and
R³⁵, R³⁶, R³⁷, R³⁸ may be the same or may be different and may be independently selected from one another from: H, halogen, nitro, cyano, trifluoromethyl, C₁-C₁₂-alkyl, 5- to 18-membered aryl, C₁-C₁₂-alkyloxy, di-(C₁-C₄-alkyl)amino, tri(C₁-C₁₂-alkyl)silyl, di(C₁-C₁₂-alkyl)(C₆-C₁₈-aryl)silyl, (C₁-C₁₂-alkyl)di(C₆-C₁₈-aryl)silyl, which in turn may be substituted with one or more of C₁-C₁₂-alkyl, 5- to 18-membered aryl, C₁-C₁₂-alkyloxy, di-(C₁-C₄-alkyl)amino, halogen, trifluoromethyl, cyano, nitro residues, which may be the same or may be different.

6. Metal organic compounds according to claim 1 or claim 2, **characterized in that** they represent compounds of the general formula 17: 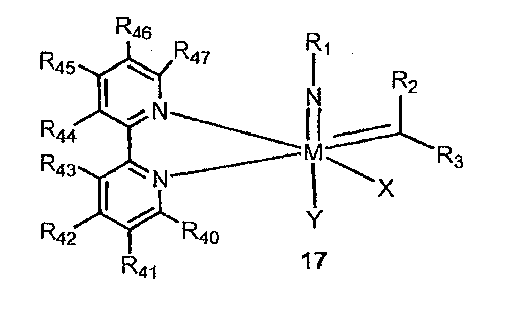
in which R¹, R², R³, X and Y are defined in claim 1 and
R⁴⁰, R⁴¹, R⁴², R⁴³, R⁴⁴, R⁴⁵, R⁴⁶, R⁴⁷ may be the same or may be different and may be independently selected from one another from H, halogen, nitro, cyano, trifluoromethyl, C₁-C₁₂-alkoxycabonyl, C₁-C₁₂-a)ky), 5- to 18-membered aryl, C₁-C₁₂-alkyloxy, di-(C₁-C₄-alkyl)amino, which in turn may be substituted with one or more of C₁-C₁₂-alkyl, 5- to 18-membered aryl, C₁-C₁₂-alkyloxy, di-(C₁-C₄-alkyl)amino, halogen, trifluoromethyl, cyano, nitro residues, which may be the same or may be different.

7. Metal organic compounds according to one of claims 1, 2 or 4, **characterized in that** they represent compounds of the general formula 24: 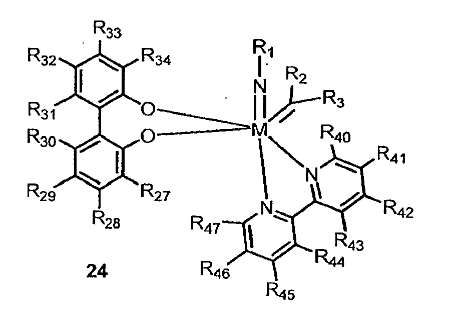 wherein
M, R¹, R² and R³ as well as R⁴⁰, R⁴¹, R⁴², R⁴³, R⁴⁴, R⁴⁵, R⁴⁶ and R⁴⁷ are defined in claims 1 and 4 and
R²⁷, R²⁸, R²⁹, R³⁰, R³¹, R³², R³³, R³⁴ may be the same or may be different and may be independently selected from one another from: H, halogen, nitro, cyano, trifluoromethyl, C₁-C₁₂-alkoxycabonyl, C₁-C₁₂-alkyl, 5- to 18-membered aryl, C₁-C₁₂-alkyloxy, di-(C₁-C₄-alkyl)amino, which in turn may be substituted with one or more of C₁-C₁₂-alkyl, 5- to 18-membered aryl, C₁-C₁₂-alkyloxy, di-(C₁-C₄-alkyl)amino, halogen, trifluoromethyl, cyano, nitro residues, which may be the same or may be different.

8. Metal organic compounds according to one of claims 1, 2, 4 or 7, **characterized in that** they represent compounds of the general formula **25:** 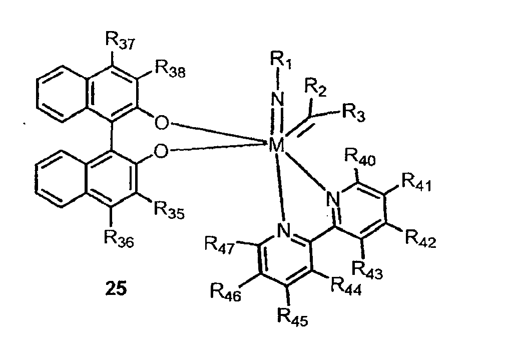 wherein
M, R¹, R² and R³ as well as R⁴⁰, R⁴¹, R⁴², R⁴³, R⁴⁴, R⁴⁵, R⁴⁶ and R⁴⁷ are defined in claims 1 and 4 and
R³⁵, R³⁶, R³⁷, R³⁸ may be the same or may be different and may be independently selected from one another from: H, halogen, nitro, cyano, trifluoromethyl, C₁-C₁₂-alkyl, 5- to 18-membered aryl, C₁-C₁₂-alkyloxy, di-(C₁-C₄-alkyl)amino, tri(C₁-C₁₂-alkyl)silyl, di(C₁-C₁₂-alkyl)(C₆-C₁₈-aryl)silyl, (C₁-C₁₂-alkyl)di(C₆-C₁₈-aryl)silyl, which in turn may be substituted with one or more of C₁-C₁₂-alkyl, 5- to 18-membered aryl, C₁-C₁₂-alkyloxy, di-(C₁-C₄-alkyl)amino, halogen, trifluoromethyl, cyano, nitro residues, which may be the same or may be different.

9. Method of preparing the metal complexes of the general formula I 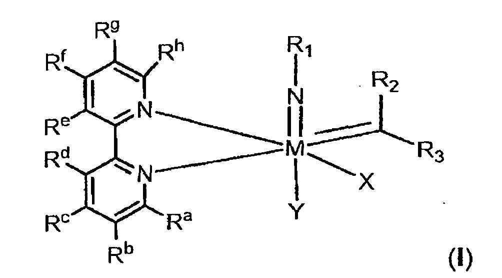 wherein
M, X, Y, R¹, R², R³, R^{a}, R^{b}, R^{c}, R^{d}, R^{e}, R^{f}, R^{g}, R^{h} are defined in claim 1,
in which a compound of formula **32** 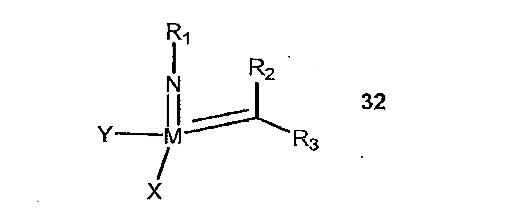 wherein M, X, Y, R₁, R₂, R₃ are defined in claim 1,
are reacted with a compound of the general formula II 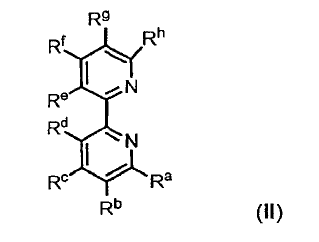 wherein R^{a}, R^{b}, R^{c}, R^{d}, R^{e}, R^{f}, R^{g}, R^{h} are defined in claim 1.

10. Use of a compound of the general formula I 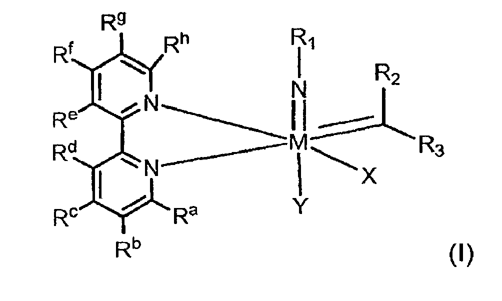 wherein M, X, Y, R¹, R², R³, R^{a}, R^{b}, R^{c}, R^{d}, R^{e}, R^{f}, R^{g}, R^{h} are defined in claim 1 as pre-catalysts for olefin metathesis reactions.

## Revendications

1. Composés organo-métalliques de la formule générale I 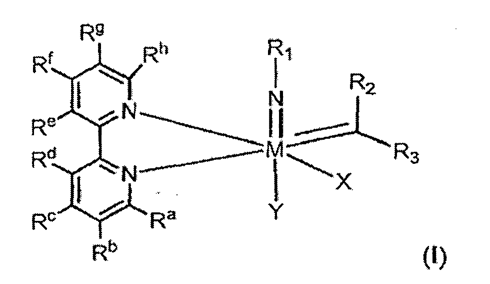 dans laquelle
M est Mo ou W,
R¹ est un groupe alkyle en C₁-C₁₂, aryle à de 5 à 18 éléments, qui peuvent être à leur tour substitués avec un ou plusieurs parmi des résidus alkyle en C₁-C₁₂, aryle à de 5 à 18 éléments, alkyloxy en C₁-C₁₂, di(alkyle en C₁-C₄)amino, halogène, trifluorométhyle, cyano, nitro, qui peuvent être identiques ou peuvent être différents,
les substituants X, Y peuvent être identiques ou peuvent être différents et peuvent être indépendamment choisis l'un de l'autre parmi : un atome d'halogène, un groupe méthanesulfonyloxy, trifluorométhanesulfonyloxy, benzènesulfonyloxy, toluènesulfonyloxy, pyrrolyle, alkyloxy en C₁-C₁₂, aryloxy à de 5 à 18 éléments, tri(alkyle en C₁-C₁₂)silyloxy, di(alkyle en C₁-C₁₂)(aryle en C₆-C₁₈)silyloxy, (alkyle en C₁-C₁₂)di(aryle en C₆-C₁₈)-silyloxy, tris(alkyloxy en C₁-C₁₂)silyloxy, qui peuvent être à leur tour substitués avec un ou plusieurs parmi des résidus alkyle en C₁-C₁₂, aryle à de 5 à 18 éléments, alcoxy en C₁-C₁₂, di-(alkyle en C₁-C₄)amino, halogène, trifluorométhyle, cyano, nitro, qui peuvent être identiques ou peuvent être différents,
R² est H, un groupe alkyle en C₁-C₁₂, aryle à de 5 à 18 éléments, qui peuvent être à leur tour substitués avec un ou plusieurs parmi des résidus alkyle en C₁-C₁₂, aryle à de 5 à 18 éléments, alkyloxy en C₁-C₁₂, di(alkyle en C₁-C₄)amino, halogène, trifluorométhyle, cyano, nitro, qui peuvent être identiques ou peuvent être différents,
R³ est un groupe alkyle en C₁-C₁₂, aryle à de 5 à 18 éléments, qui peuvent être à leur tour substitués avec un ou plusieurs parmi des résidus alkyle en C₁-C₁₂, aryle à de 5 à 18 éléments, alkyloxy en C₁-C₁₂, di(alkyle en C₁-C₄)amino, halogène, trifluorométhyle, cyano, nitro, qui peuvent être identiques ou peuvent être différents,
R^{a}, R^{b}, R^{c}, R^{d}, R^{e}, R^{f}, R^{g}, R^{h} peuvent être identiques ou peuvent être différents et peuvent être indépendamment choisis l'un de l'autre parmi H, un atome d'halogène, un groupe nitro, cyano, trifluorométhyle, alcoxycarbonyle en C₁-C₁₂, alkyle en C₁-C₁₂, aryle à de 5 à 18 éléments, alkyloxy en C₁-C₁₂, di-(alkyle en C₁-C₄)amino, qui peuvent être à leur tour substitués avec un ou plusieurs parmi des résidus alkyle en C₁-C₁₂, aryle à de 5 à 18 éléments, alkyloxy en C₁-C₁₂, di-(alkyle en C₁-C₄)amino, halogène, trifluorométhyle, cyano, nitro, qui peuvent être identiques ou peuvent être différents, ou les résidus R^{d} et R^{e} sont connectés les uns aux autres tout en formant un cycle à de 5 à 8 éléments.

2. Complexes métalliques selon la revendication 1, **caractérisés en ce que** R^{d} et R^{e} sont un résidu R⁴⁸, respectivement, qui est CR⁴⁹R⁵⁰, CR⁵¹=CR⁵², CR⁵³R⁵⁴-CR⁵⁵R⁵⁶, CR⁵⁷R⁵⁸-CR⁵⁹R⁶⁰-CR⁶¹R⁶², CR⁶²R⁶³=CR⁶⁴R⁶⁵R⁶⁷, CR⁶⁹R⁷⁰-CR⁷¹R⁷²-CR⁷³R⁷⁴-CR⁷⁵CR⁷⁶, CR⁷⁷=CR⁷⁸-CR⁷⁹R⁸⁰-CR⁸¹CR⁸² et/ou CR⁸³R⁸⁴-CR⁸⁵=CR⁸⁷-CR⁸⁸CR⁸⁹ où R⁴⁹, R⁵⁰, R⁵¹, R⁵², R⁵³, R⁵⁴, R⁵⁵, R⁵⁶, R⁵⁷, R⁵⁸, R⁵⁹, R⁶⁰, R⁶¹, R⁶², R⁶³, R⁶⁴, R⁶⁵, R⁶⁶, R⁶⁷, R⁶⁸, R⁶⁹, R⁷⁰, R⁷¹, R⁷², R⁷³, R⁷⁴, R⁷⁵, R⁷⁶, R⁷⁷, R⁷⁸, R⁷⁹, R⁸⁰, R⁸¹, R⁸², R⁸³, R⁸⁴, R⁸¹, R⁸⁶, R⁸⁷, R⁸⁸ et R⁸⁹ sont indépendamment choisis l'un de l'autre, respectivement, et peuvent présenter la même signification que R^{a}.

3. Complexes organo-métalliques selon la revendication 1 ou la revendication 2, **caractérisés en ce qu'**ils représentent des composés de la formule générale 2 : 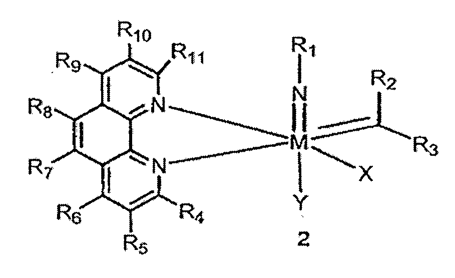 dans laquelle
R¹, R², R³, X et Y sont définis dans la revendication 1, et
R⁴, R⁵, R⁶, R⁷, R⁸, R⁹, R¹⁰ R¹¹ peuvent être identiques ou peuvent être différents et peuvent être indépendamment choisis l'un de l'autre parmi H, un atome d'halogène, un groupe nitro, cyano, trifluorométhyle, alcoxycarbonyle en C₁-C₁₂, alkyle en C₁-C₁₂, aryle à de 5 à 18 éléments, alkyloxy en C₁-C₁₂, di-(alkyle en C₁-C₄)amino, qui peuvent à leur tour être substitués avec un ou plusieurs parmi des résidus alkyle en C₁-C₁₂, aryle à de 5 à 18 éléments, alkyloxy en C₁-C₁₂, di-(alkyle en C₁-C₄)amino, halogène, trifluorométhyle, cyano, nitro, qui peuvent être identiques ou peuvent être différents.

4. Composés organo-métalliques selon l'une quelconque des revendications 1 à 3, **caractérisés en ce qu'**ils représentent des composés de la formule générale 9 : 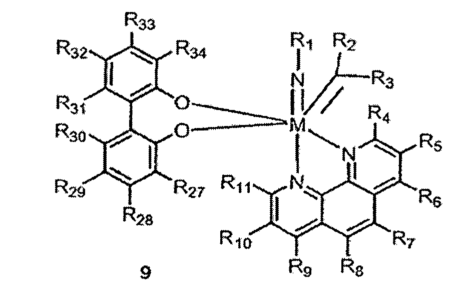 dans laquelle
M, R¹, R², R³ et R⁴, R⁵, R⁶, R⁷, R⁸, R⁹, R¹⁰, R¹¹ sont définis ci-dessus,
R²⁷, R²⁸, R²⁹, R³⁰, R³¹, R³², R³³, R³⁴ peuvent être identiques ou peuvent être différents et peuvent être indépendamment choisis l'un de l'autre parmi : H, un atome d'halogène, un groupe nitro, cyano, trifluorométhyle, alcoxycarbonyle en C₁-C₁₂, alkyle en C₁-C₁₂, aryle à de 5 à 18 éléments, alkyloxy en C₁-C₁₂, di-(alkyle en C₁-C₄)amino, qui peuvent à leur tour être substitués avec un ou plusieurs parmi des résidus alkyle en C₁-C₁₂, aryle à de 5 à 18 éléments, alkyloxy en C₁-C₁₂, di-(alkyle en C₁-C₄) amino, halogène, trifluorométhyle, cyano, nitro, qui peuvent être identiques ou peuvent être différents.

5. Composés organo-métalliques selon l'une quelconque des revendications 1 à 4, **caractérisés en ce qu'**ils représentent des composés de la formule générale 10 : 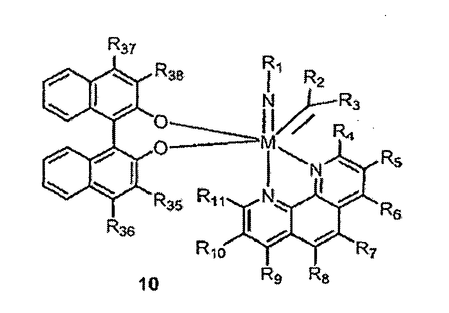 dans laquelle
M, R¹, R², R³, R⁴, R⁵, R⁶, R⁷, R⁸, R⁹, R¹⁰ et R¹¹ sont définis dans les revendications 1 et 3 et R³⁵, R³⁶, R³⁷, R³⁸ peuvent être identiques ou peuvent être différents et peuvent être indépendamment choisis l'un de l'autre parmi : H, un atome d'halogène, un groupe nitro, cyano, trifluorométhyle, alkyle en C₁-C₁₂, aryle à de 5 à 18 éléments, alkyloxy en C₁-C₁₂, di-(alkyle en C₁-C₄)amino, tri(alkyle en C₁-C₁₂)silyle, di(alkyle en C₁-C₁₂)(aryle en C₆-C₁₈)silyle, (alkyle en C₁-C₁₂)di(aryle en C₆-C₁₈)silyle, qui peuvent à leur tour être substitués avec un ou plusieurs parmi des résidus alkyle en C₁-C₁₂, aryle à de 5 à 18 éléments, alkyloxy en C₁-C₁₂, di-(alkyle en C₁-C₄)amino, halogène, trifluorométhyle, cyano, nitro, qui peuvent être identiques ou peuvent être différents.

6. Composés organo-métalliques selon la revendication 1 ou la revendication 2, **caractérisés en ce qu'**ils représentent des composés de la formule générale 17 : 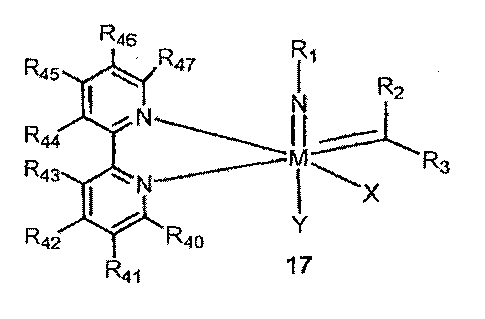
dans laquelle R¹, R², R³, X et Y sont définis dans la revendication 1 et
R⁴⁰, R⁴¹, R⁴², R⁴³, R⁴⁴, R⁴⁵, R⁴⁶, R⁴⁷ peuvent être identiques ou peuvent être différents et peuvent être indépendamment choisis l'un de l'autre parmi H, un atome d'halogène, un groupe nitro, cyano, trifluorométhyle, alcoxycarbonyle en C₁-C₁₂, alkyle en C₁-C₁₂, aryle à de 5 à 18 éléments, alkyloxy en C₁-C₁₂, di-(alkyle en C₁-C₄)amino, qui peuvent être à leur tour substitués avec un ou plusieurs parmi des résidus alkyle en C₁-C₁₂, aryle à de 5 à 18 éléments, alkyloxy en C₁-C₁₂, di-(alkyle en C₁-C₄)amino, halogène, trifluorométhyle, cyano, nitro, qui peuvent être identiques ou peuvent être différents.

7. Composés organo-métalliques selon l'une quelconque des revendications 1, 2 ou 4, **caractérisés en ce qu'**ils représentent des composés de la formule générale 24 : 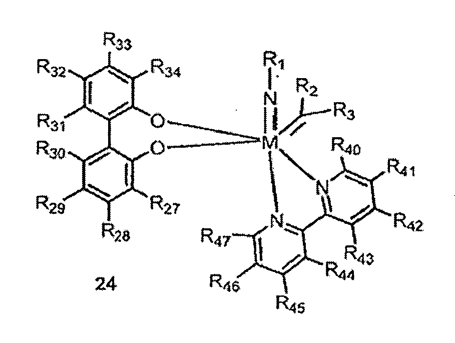 dans laquelle
M, R¹, R² et R³ ainsi que R⁴⁰, R⁴¹, R⁴², R⁴³, R⁴⁴, R⁴⁵, R⁴⁶ et R⁴⁷ sont définis dans les revendications 1 et 4 et
R²⁷, R²⁸, R²⁹, R³⁰, R³¹, R³², R³³, R³⁴ peuvent être identiques ou peuvent être différents et peuvent être indépendamment choisis l'un de l'autre parmi : H, un atome d'halogène, un groupe nitro, cyano, trifluorométhyle, alcoxycarbonyle en C₁-C₁₂, alkyle en C₁-C₁₂, aryle à de 5 à 18 éléments, alkyloxy en C₁-C₁₂, di-(alkyle en C₁-C₄)amino, qui peuvent à leur tour être substitués avec un ou plusieurs parmi des résidus alkyle en C₁-C₁₂, aryle à de 5 à 18 éléments, alkyloxy en C₁-C₁₂, di-(alkyle en C₁-C₄)amino, halogène, trifluorométhyle, cyano, nitro, qui peuvent être identiques ou peuvent être différents.

8. Composés organo-métalliques selon l'une quelconque des revendications 1, 2, 4 ou 7, **caractérisés en ce qu'**ils représentent des composés de la formule générale 25 : 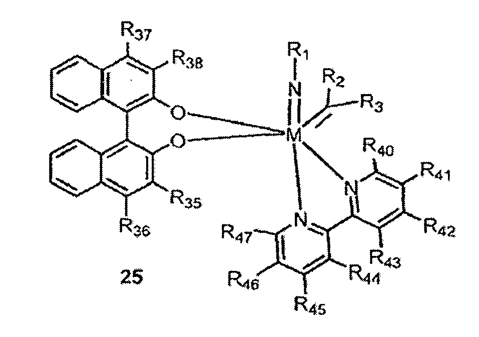 dans laquelle
M, R¹, R² et R³ ainsi que R⁴⁰, R⁴¹, R⁴², R⁴³, R⁴⁴, R⁴⁵, R⁴⁶ et R⁴⁷ sont définis dans les revendications 1 et 4 et
R³⁵, R³⁶, R³⁷, R³⁸ peuvent être identiques ou peuvent être différents et peuvent être indépendamment choisis l'un de l'autre parmi : H, un atome d'halogène, un groupe nitro, cyano, trifluorométhyle, alkyle en C₁-C₁₂, aryle à de 5 à 18 éléments, alkyloxy en C₁-C₁₂, di-(alkyle en C₁-C₄)amino, tri(alkyle en C₁-C₁₂)silyle, di(alkyle en C₁-C₁₂)(aryle en C₆-C₁₈) silyle, (alkyle en C₁-C₁₂)di(aryle en C₆-C₁₈)silyle, qui peuvent être à leur tour substitués avec un ou plusieurs parmi des résidus alkyle en C₁-C₁₂, aryle à de 5 à 18 éléments, alkyloxy en C₁-C₁₂, di-(alkyle en C₁-C₄)amino, halogène, trifluorométhyle, cyano, nitro, qui peuvent être identiques ou peuvent être différents.

9. Procédé de préparation des complexes métalliques de la formule générale I 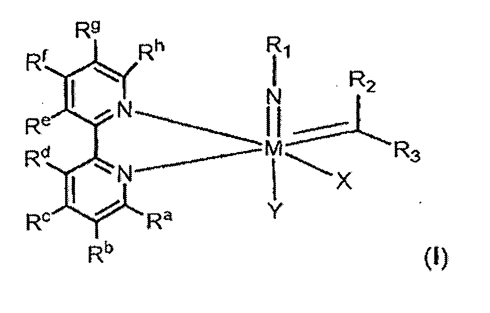 dans laquelle
M, X, Y, R¹, R², R³, R^{a}, R^{b}, R^{c}, R^{d}, R^{e}, R^{f}, R^{g}, R^{h} sont définis dans la revendication 1, dans lequel un composé de formule 32 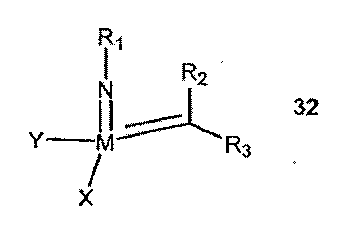
dans laquelle M, X, Y, R₁, R₂, R₃ sont définis dans la revendication 1,
réagit avec un composé de la formule générale II 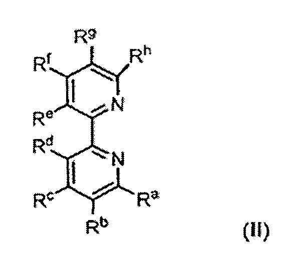 dans laquelle R^{a}, R^{b}, R^{c}, R^{d}, R^{e}, R^{f}, R^{g}, R^{h} sont définis dans la revendication 1.

10. Utilisation d'un composé de la formule générale I 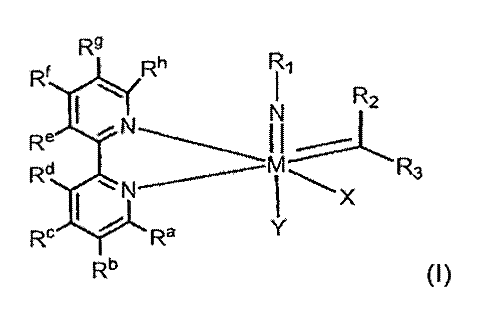 dans laquelle M, X, Y, R¹, R², R³, R^{a}, R^{b}, R^{c}, R^{d}, R^{e}, R^{f}, R^{g}, R^{h} sont définis dans la revendication 1 comme pré-catalyseurs pour des réactions de métathèses d'oléfines.
